# EUROPEAN PATENT APPLICATION

(11) **EP 4 442 272 A1**
(43) Date of publication of application: **09.10.2024**
(21) Application number: 22913401.0
(22) Date of filing: 17.08.2022
(51) Int. Cl.: A61K 39/39, A61K 39/25, A61K 39/12, A61P 31/22, A61P 35/00

(54) **VACCINE ADJUVANT, AND PREPARATION METHOD THEREFOR AND USE THEREOF**

(30) Priority: 28.12.2021 CN 202111627083; 28.12.2021 CN 202111627088; 06.04.2022 CN 202210358103
(71) Applicant: Chengdu Maxvax Biotechnology LLC, Chengdu, Sichuan 610041 (CN)
(72) Inventor: CHEN, Dexiang, Chengdu, Sichuan 610041 (CN); HU, Yeqin, Chengdu, Sichuan 610041 (CN); SUI, Qiang, Chengdu, Sichuan 610041 (CN); AI, Xulu, Chengdu, Sichuan 610041 (CN); FANG, Xijing, Chengdu, Sichuan 610041 (CN)
(74) Representative: Kransell & Wennborg KB
(86) International application number: PCT/CN2022/113087
(87) International publication number: WO 2023/124116

(57) **Abstract**

A vaccine adjuvant, and a preparation method therefor and a use thereof. The vaccine adjuvant is a MA105 immunologic adjuvant, and comprises (1) QS-21: 50 µg/ml to 300 µg/ml; (2) Poly I:C: 400 µg/mL to 3000 µg/mL; and (3) lipid molecules constituting a vector, the vector being a mixture of a cationic liposome and a neutral liposome.

## Description

### TECHNICAL FIELD

The present invention belongs to the field of medical biotechnologies, and specifically relates to a vaccine adjuvant, a preparation method therefor and use thereof.

### BACKGROUND ART

### 1. Immunologic Adjuvant

In immunology, an adjuvant is an ingredient capable of enhancing immune responses to an antigen and/or regulating immune responses. An immunologic adjuvant is defined as any substance capable of accelerating, prolonging or enhancing antigen-specific immune responses when being bound to a particular vaccine antigen. Many known adjuvants are widely used, including oil, aluminum salts and viroids. It is generally believed that adjuvants that activate immunity of cells (particularly CD4+ T cells) can produce more effective therapeutic effects.

### 1.1. QS-21 Adjuvant

Quillaja saponaria saponin 21 (QS-21) has a following structural formula, with a molecular formula C92H148O46 and a molecular weight 1990.14, and is generally a colorless transparent liquid.

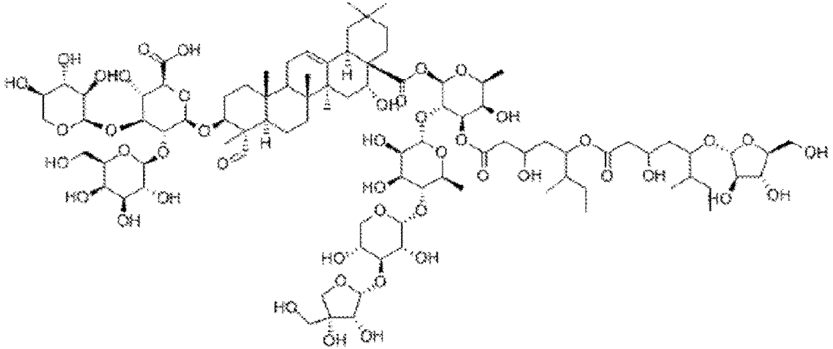

In population of cancer patients, QS-21 dose within a range of 100-200 µg has been demonstrated to have optimal activity and good tolerance. Reactogenicity within this dose range is that most patients have 2-10 cm erythema and induration at the injection site, and occasionally have mild low-grade flu-like symptoms. No dose-limiting toxicity within this dose range has been reported. QS-21 is a novel medicinal adjuvant with high safety and good tolerance.

### 1.2. Liposomes

Liposomes are an artificial cell membrane-like globule composed of lipids, of a monolayer or multilayer bimolecular vesicle structure mainly composed of phosphate lipids, cholesterol, and stearylamine, can be used to encapsulate many kinds of vaccine antigens, and are effectively introduced into cells. Liposomes tend to deposit *in vivo* in the reticuloendothelial phagocyte system such as the liver, spleen, and lymph node, prolong residence time *in vivo*, and has the effect of reducing the dosage of vaccine, reducing toxic side effects, and improving immunity. Liposomes have a significant adjuvant effect for a variety of antigens.

### 1.3. Immunologic Adjuvant and Tumor Immunotherapy

Immunological therapy (including immune checkpoint inhibitor antibody, immunotherapy, anti-tumor vaccine and so on) is currently being assessed intensively for the development of clinical cancer treatments. Current cancer immunological therapy still has many limitations, such as low tumor vaccine release efficiency, blocked cross presentation of tumor tissue-specific antigens, and effects of a series of immunosuppressive cytokines.

In the development of tumor vaccine products, immunological adjuvants play a critical role in improving immunogenicity of tumor antigens and activating adaptive antigen-specific immune responses. As a substance improving the adaptive immune responses to antigens, adjuvants can address the problem of poor immunogenicity of tumor antigens, enhance immune responses to tumor antigens, effectively avoid immune tolerance induced and generated after immature antigen presenting cell (APC) contact with antigens, and improve immunotherapeutic efficacy of tumor vaccines.

Therefore, the study of adjuvants in tumor immunotherapy has been rapidly explored and developed. In addition to conventional aluminum salt adjuvants, new adjuvants such as pattern recognition receptor agonists (such as Toll-like receptor agonists and C-type lectin receptor agonists), polymeric materials and polypeptides have all been found to have good immune activation effects.

### 2. Herpes Zoster Vaccine

In 2017, the Advisory Committee on Immunization Practices (ACIP) of the US Centers for Disease Control and Prevention (CDC) issued opinions recommending replacing Merck's attenuated live vaccine Zostavax^{®} with GlaxoSmithKline's recombinant, adjuvanted subunit vaccine Shingrix^{®} for vaccination of adults aged 50 and above.

The protective efficacy of the live attenuated varicella zoster vaccine Zostavax^{®} produced by Merck gradually decreases with the increase of age of vaccinees, and Phase III clinical trial results showed that the protective rate against herpes zoster is about 64% in the population aged 60-69, while the protective rate declines to 38% in the population aged more than 70. Moreover, this vaccine cannot be used for or produce effective protection in populations with immune system deficiencies. Shingrix^{®}, produced by GSK, is a recombinant subunit vaccine, whose technical core is derived from its AS01_{B} adjuvant system and specific antigen, and phase III clinical trial results show that this vaccine has an overall efficacy against herpes zoster of 97.2%, and also achieved 97.5% protective efficacy in the population aged 70 or more. Moreover, it was found that the number of antigen-specific CD4⁺ T cells induced by Shingrix^{®} is approximately ten times the number of CD4⁺ T cells induced by Zostavax^{®}.

Although aluminum adjuvants are commonly used in vaccine formulations, studies show that the aluminum adjuvants mainly enhance antibodies and Th2 cell-mediated humoral immune responses, and do not enhance Th1 cell-mediated cellular immune responses (which includes induced expression of cytokines such as IL-2 and IFN-γ). Inactivated varicella zoster vaccine formulated by Merck with aluminum adjuvant failed to be marketed because its immunogenicity did not meet expectations, which further indicates that adjuvants capable of inducing immune responses of cells (particularly CD4⁺ T cells) is a key factor for high protection of varicella zoster vaccines.

Based on this, the present invention relates to a novel adjuvant system, termed MA105, and a preparation method therefor, and use of the adjuvant in the preparation of a therapeutic product. The therapeutic product includes:
(1) a recombinant herpes zoster vaccine (CHO cell) preparation; and
(2) an immunologic adjuvant that activates tumor immunity.

### SUMMARY

The present invention first relates to an MA105 immunologic adjuvant, which contains:
(1) QS-21, 50-300 µg/ml;
(2) Poly I:C, 400-3000 µg/ml; and
(3) lipid molecules constituting a common delivery system for immunostimulatory molecules and antigens.

The delivery system is liposomes, which may be cationic liposomes, neutral liposomes or a mixture of cationic and neutral liposomes.

Preferably, the delivery system is a mixture of cationic and neutral liposomes.

Preferably, the MA105 adjuvant system contains:
(1) QS-21, 50-200 µg/ml; and
(2) Poly I:C, 400-3000 µg/ml.

The lipid molecules are:
(3) DOTAP, 70-560 µg/ml;
(4) DOPC, 500-4000 µg/ml; and
(5) cholesterol, 175-1400 µg/ml.

More preferably, the MA105 adjuvant comprises:
(1) QS-21, 80-120 µg/ml; and
(2) Poly I:C, 780-1600 µg/ml.

The lipid molecules are:
(3) DOTAP, 140-280 µg/ml;
(4) DOPC, 1200-2500 µg/ml; and
(5) cholesterol, 350-600 µg/ml.

The present invention further relates to a preparation method for the MA105 adjuvant, which includes the following steps:
(1) preparing cationic liposomes and neutral liposomes, respectively; and
(2) mixing QS-21, Poly I:C, the cationic liposomes, and the neutral liposomes and stirring to obtain the MA105 adjuvant system.

A preparation method for the cationic liposomes is:
(1) formulating 1,2-dioleoyl-3-trimethyl ammonium chloride propane (DOTAP), dioleoyl phosphatidylcholine (DOPC) and cholesterol according to a mass ratio of 2:2: 1; and
(2) dispersing the three lipid ingredients uniformly, and then preparing the liposomes by an ethanol injection method, a film dispersion method, an ultrasonic dispersion method, and a reverse evaporation method.

Preferably, the cationic liposomes are prepared by the ethanol injection method, and the preparation method is as follows: dispersing the three lipid ingredients uniformly and then preparing primary emulsion in a water phase, after being subjected to an ultrafiltration replacement buffer solution, extruding and molding by extrusion membranes having a pore size of 200 nm and 100 nm in turn; and finally sterilizing by filtration to obtain the cationic liposomes.

More preferably, preparation steps of the cationic liposomes are as follows:
(1) dissolving the three lipid ingredients in ethanol, and shearing on line with water phase for 1-3 times;
(2) carrying out ultrafiltration replacement using a sucrose-containing buffer solution, concentration multiple being 2-4 and washing-filtering multiple being 6-8;
(3) extruding by a filter membrane with a pore size of 200 nm, cyclically performing multiple rounds of extrusion, and controlling particle size homogeneity;
(4) extruding by a filter membrane with a pore size of 100 nm, cyclically performing multiple rounds of extrusion, and controlling particle size homogeneity; and
(5) Sterile filtering using a filter.

Physical and chemical parameters of the cationic liposome are:
average particle size (D50): 30-90 nm;
solution pH: 4.8-7.0, preferably 4.8-6.0; and
Zeta potential: 40-72 mV (pH 6.5).

A preparation method for the neutral liposomes is:
(1) formulating DOPC and cholesterol according to a mass ratio of 4:1; and
(2) dispersing lipid ingredients uniformly, and then preparing the liposomes by an ethanol injection method, a film dispersion method, an ultrasonic dispersion method, and a reverse evaporation method.

Preferably, the neutral liposomes are prepared by the ethanol injection method, and a preparation method is as follows: dispersing the lipid ingredients uniformly and then preparing primary emulsion in the water phase, extruding and molding by extrusion membranes having a pore size of 200 nm and 100 nm in turn, subsequently being subjected to an ultrafiltration replacement buffer solution, and then filtering and sterilizing to obtain the neutral liposomes.

More preferably, preparation steps of the neutral liposomes are as follows:
(1) dissolving two lipid ingredients in ethanol, and shearing on line with water phase for 1-3 times;
(2) filtering by a filter membrane with a pore size of 200 nm, cyclically performing multiple rounds of extrusion, and controlling particle size homogeneity;
(3) filtering by a filter membrane with a pore size of 100 nm, cyclically performing multiple rounds of extrusion, and controlling particle size homogeneity;
(4) buffer replacement by ultrafiltration using a sucrose-containing buffer solution, concentration 3-6 times and washing-filtering 6-8 times; and
(5) sterilizing by filtration.

Physical and chemical parameters of the neutral liposomes are as follows:
average particle size (D50): 80-140 nm; and
solution pH: 5.5-7.0, preferably 6.4-6.6.

The MA105 adjuvant system contains two immunopotentiating agents (QS-21 and poly (I:C)). In order to reduce toxicity of QS-21 and Poly I:C and improve immunogenicity, the mixture of the cationic liposomes and the neutral liposomes is used to adsorb QS-21 and Poly I:C, or mixed liposomes of the neutral liposomes having adsorbed QS-21 and the cationic liposomes are used to further adsorb the poly (I:C). Signal transduction of poly (I:C) mainly relies on Toll-like receptor 3 (TLR3) and melanoma differentiation-associated gene 5 (MDA-5), can strongly drive cellular immunity and effective type I interferon responses, and can induce strong antigen-specific CD4⁺ and CD8⁺ T-cell responses through type I interferon (IFN-α/β) signal transduction and antigen cross presentation. We use the cationic liposomes (consisting of DOTAP, DOPC and cholesterol) as the delivery system to adsorb poly (I:C) by electrostatic interaction.

The present invention further relates to use of the MA105 adjuvant system in the preparation of a vaccine formulation, wherein the vaccine formulation contains a therapeutically effective amount of antigen, the MA105 adjuvant system and indispensable pharmaceutical auxiliary materials. Preferably, the vaccine formulation is a recombinant herpes zoster vaccine preparation.

The pharmaceutical auxiliary materials include, but are not limited to, polysorbate 80, histidine, sodium dihydrogen phosphate, sucrose, and sodium chloride.

The present invention further relates to a method for preparing a recombinant herpes zoster vaccine preparation using the MA105 adjuvant system, wherein the method includes the following steps:
(1) to a purified antigen solution adding sucrose, histidine and Tween-80 according to a formula of the antigen buffer solution, and regulating pH to obtain a primary antigen liquid,
(2) mixing the primary antigen liquid and the MA105 adjuvant system and stirring uniformly to obtain a semi-finished product; and
(3) subpackaging the semi-finished product to obtain a finished product of recombinant herpes zoster vaccine preparation.

The primary antigen liquid contains a herpes zoster gE protein antigen having a sequence set forth in SEQ ID NO. 1 (wherein molecular weight is 63530.6, and isoelectric point is 4.8-5.8), wherein

SEQ ID NO. 1:
SVLRYDDFHTDEDKLDTNSVYEPYYHSDHAESSWVNRGESSRKAYDHNSPYIWPRNDYDGFLE
NAHEHHGVYNQGRGIDSGERLMQPTQMSAQEDLGDDTGIHVIPTLNGDDRHKIVNVDQRQYGDVFK
GDLNPKPQGQRLIEVSVEENHPFTLRAPIQRIYGVRYTETWSFLPSLTCTGDAAPAIQHICLKHTTCFQD
VVVDVDCAENTKEDQLAEISYRFQGKKEADQPWIVVNTSTLFDELELDPPEIEPGVLKVLRTEKQYLG
VYIWNMRGSDGTSTYATFLVTWKGDEKTRNPTPAVTPQPRGAEFHMWNYHSHVFSVGDTFSLAMHL
QYKIHEAPFDLLLEWLYVPIDPTCQPMRLYSTCLYHPNAPQCLSHMNSGCTFTSPHLAQRVASTVYQN
CEHADNYTAYCLGISHMEPSFGLILHDGGTTLKFVDTPESLSGLYVFVVYFNGHVEAVAYTVVSTVDH
FVNAIEERGFPPTAGQPPATTKPKEITPVNPGTSPLLRY.

Preferably, in terms of per milliliter of the vaccine preparation, the recombinant herpes zoster vaccine preparation prepared with an MA105 adjuvant system contains:
(1) the gE protein antigen having a sequence set forth in SEQ ID NO. 1, with a content of 95-110 µg/ml;
(2) a pharmaceutical auxiliary material combination for maintaining stability of the gE protein antigen and liposome, the pharmaceutical auxiliary material combination containing:
   1) polysorbate 80, 450-550 µg/ml;
   2) histidine, 200-950 µg/ml; and
   3) sucrose, 52-55 mg/ml;
(3) an MA105 immunologic adjuvant system, containing:
   1) DOTAP, 140-215 µg/ml;
   2) DOPC, 1200-2200 µg/ml;
   2) cholesterol, 320-550 µg/ml;
   3) QS-21, 80-110 µg/ml; and
   4) Poly I:C, 780-830 µg/ml; and
(4) other pharmaceutical auxiliary materials, containing:
   1) sodium dihydrogen phosphate, 0-800 µg/ml; and
   2) sodium chloride, 2.5-6 mg/ml.

Further,
an osmotic pressure of the vaccine preparation is 210-350 mOsmol/kg;
an average particle size of the vaccine preparation is 100-300 nm, preferably 130-200 nm; and
pH of the vaccine preparation is 6.0-7.0.

The present invention further relates to a recombinant herpes zoster vaccine preparation prepared by the method.

The present invention further relates to following use of the recombinant herpes zoster vaccine preparation: treatment or prevention of diseases caused by varicella zoster virus (VZV), wherein the treatment is single treatment or combined treatment with other drugs or vaccines.

The present invention further relates to an anti-tumor vaccine product, wherein the anti-tumor vaccine product contains:
(1) an effective amount of tumor antigen, the tumor antigen being a recombinant polypeptide antigen;
(2) the MA105 adjuvant system; and
(3) indispensable pharmaceutical auxiliary materials.

Preferably, the anti-tumor vaccine product is administered by subcutaneous or intramuscular injection.

Preferably, the tumor antigen is an HPV antigen having an amino acid sequence set forth in SEQ ID NO. 2 or an HPV antigen having an amino acid sequence set forth in SEQ ID NO. 3, wherein
SEQ ID NO. 2: 
HQKRTAMFQDPQERPRKLPQLCTELQTTIHDIILECVYCKQQLLREVYDFAFRDLCIVYRDGNPY
AVGDKCLKFYSKVSEYRYYCYSLYGTTLEQQYNKPLCDLLIRGINCQKPLCPDEKQRHLDKKQRFHNI
RGRWTGRCMSCCRSSRTRRETQLgsgsgsgsgsgMHGDTPTLHEYMLDLQPETTDLYGYGQLHDSSEEE
DEIDGPAGQAEPDRAHYNIVTFCCKCDSTLRLCVQSTHVDIRTLEDLLMGTLGIVGPICSQKP
SEQ ID NO. 3:

ARFEDPTRRPYKLPDLCTELNTSLQDIEITCVYCKTVLELTEVFEFAFKDLFVVYRDSIPHAAGHK
CIDFYSRIRELRHYSDSVYGDTLEKLTNTGLYNLLIRGLRCQKPLNPAEKLRHLNEKRRFHNIAGHYRG
QCHSCCNRARQERLQRRRETQVgsgsgsgsgsgMHGPKATLQDIVLHLEPQNEIPVDLLGHGQLSDSEEE
NDEIDGVNHQHLPARRAEPQRHTMLCMCCKCEARIELVVESSADDLRAFQQLFLNTLSFVGPWCASQ
Q.

Preferably, the pharmaceutical auxiliary materials include: a stabilizer, a freeze-thawing protective agent, and an osmotic pressure regulator.

More preferably, the pharmaceutical auxiliary materials include: polysorbate 80, sucrose, and sodium chloride.

The present invention further relates to an anti-tumor preparation, containing:
(1) an effective amount of MA105 adjuvant system; and
(2) indispensable pharmaceutical auxiliary materials.

Preferably, the anti-tumor preparation is an intratumoral injection preparation or a tumor in-situ vaccine.

Preferably, the tumor type is melanoma, colorectal cancer or lung cancer.

Preferably, the pharmaceutical auxiliary materials include: a stabilizer, a freeze-thawing protective agent, and an osmotic pressure regulator.

More preferably, the pharmaceutical auxiliary materials include: polysorbate 80, sucrose, and sodium chloride, histidine, and sodium dihydrogen phosphate.

The present invention further relates to use of the MA105 adjuvant system or a preparation containing the MA105 adjuvant system in the preparation of an anti-tumor combined preparation, wherein the combined preparation contains a therapeutically effective amount of the MA105 adjuvant system and any other drug selected from the group consisting of:
(1) a cytotoxic chemotherapeutic drug;
(2) an antibody anti-tumor drug;
(3) an oncolytic peptide and oncolytic virus drug; and
(4) a cell therapy preparation, preferably Car-T cell therapy preparation.

When used as a therapy preparation, the MA105 adjuvant system or the vaccine composition containing the MA105 adjuvant system in the present invention may be administered orally or parenterally. In the case of parenteral administration, the adjuvant system or vaccine composition may be administered by means of in situ injection, focal in situ injection, intravenous injection, subcutaneous injection, intramuscular injection, intraperitoneal injection, transdermal administration, and the like.

When an antigen-free MA105 adjuvant system in the present invention or a tumor in situ vaccine prepared with the MA105 adjuvant system is administered, immune protection in a subject is activated by means of intratumoral injection.

The present invention further relates to a preparation method for preparing Quillaja saponaria saponin 21 (QS21) immunologic adjuvant by taking semi-purified saponin of commercial grade or food additive grade as raw material, wherein the method includes the following steps:
(1) saponin dissolution and filtration,
   dissolving the saponin raw material in a diluent, and then removing undissolved substances using a filter;
(2) reverse phase rough purification,
   carrying out reverse phase rough purification on a saponin filtered sample solution obtained in step (1) according to the following steps:
   1) loading: loading on a UniPSN 30-300 chromatographic medium; and
   2) elution: performing linear gradient elution of target peak with 2.5% B to 21.25±5.00% B, periodically collecting high-purity samples, and merging roughly purified saponin solution;
(3) reverse phase fine purification,
   carrying out reverse phase fine purification on roughly purified saponin solution obtained in step (2) according to the following steps:
   1) loading: diluting sample solution in water to acetonitrile concentration of 22-28%, and loading on a UniPS 10-300 chromatographic medium;
   2) elution: performing linear gradient elution of target peak with 10% B to 25±5% B, periodically collecting high-purity samples, and merging; and
   3) making merged samples repeat the above 1)-2) steps for 1-3 times, preferably 2 times;
(4) step of precipitation and re-dissolution,
   1) to purified saponin sample solution prepared in step (3), adding water of 2 times volume and uniformly mixing them, placing at room temperature, then centrifuging and collecting precipitate; and
   2) to the precipitate adding a dissolving solution, stirring and dissolving, to obtain sample with organic solvent being removed, preferably, an amount of the dissolving solution added being the same as that of the purified saponin sample solution prepared in step (3); and
(5) step of ultrafiltration replacement,
   1) ultrafiltration-concentrating the saponin sample solution obtained after precipitation and re-dissolution in step (4) with an ultrafiltration replacement solution, to obtain concentrated QS21 adjuvant solution, preferably, ultrafiltration-concentrating to 30-50% of the original volume.

In the above steps (1)-(5),
the diluent is a water solution containing 30% acetonitrile and 5 mM citric acid, with pH 5.0;
B solution is a water solution containing 99% acetonitrile and 0.1% TFA;
the dissolving solution is a water solution containing 5 mM histidine, with pH 6.0; and
the ultrafiltration replacement solution is a water solution containing 5 mM histidine, with pH 5.0.

In the saponin raw material, QS21 content is not lower than 8%, moisture content is not higher than 10%, and ash content is not higher than 3%.

Yield of QS21 purified by the method is not lower than 50%, and purity of finally obtained QS21 is not lower than 94%.

Further,
in step (1), the saponin raw material is dissolved 1:25 (W/V), and then filtered by a 1.0 + 0.45 µm filter;
in step (2), a loading amount is 30-60 mg/ml, a linear flow rate is 90-360 cm/h, after finishing the loading, A1 solution is used for rinsing, and impurities are rinsed with 2.5% B solution; and in the gradient elution, samples with purity higher than 30% are collected and merged;
in step (3), a loading amount is 10-50 mg/ml, after finishing the loading, A2 solution is used for rinsing, and impurities are rinsed with 10% B, samples with purity higher than 60% are collected in first reverse phase fine purification, samples with purity higher than 80% are collected in second reverse phase fine purification, samples with purity higher than 90% are collected in third reverse phase fine purification, and samples with purity higher than 95% are collected in fourth reverse phase fine purification;
in step (4), mixture is placed at room temperature for not less than 30 min, and centrifuging parameters are 9000-11500 g, 2-8 °C for 10 min; and
in step (5), ultrafiltration parameters are: using 5kD membrane package, transmembrane pressure of 0.2-0.4 bar, 10 times of replacement, and washing-filtering with the ultrafiltration replacement solution for not less than 8 times volume.

In the above,
the A1 solution is a water solution containing 30% acetonitrile and 0.1% TFA; and
the A2 solution is a water solution containing 20% acetonitrile and 0.1% TFA.

The present invention has the following beneficial effects.
1. The MA105 adjuvant system includes the mixed liposomes and two immunopotentiating agents poly (I:C) and QS-21, which has good safety, and can induce the generation of a high level of antibodies and a high frequency of CD4⁺ T cells.

1. When the MA105 adjuvant system is administered alone, it is administered by intratumoral injection, which can stimulate immune cells in the tumor, especially tumor-killing immune cells, activate tumor immunity, and thus achieve the effect of treating the tumor.
2. When the MA105 adjuvant system is used with a tumor antigen, only a low dose of antigen is required to stimulate the body to generate specific cellular immunity and generate memory immune protection.

### Definitions and Description of Terms

Unless indicated otherwise, the terms used in the present invention have the meanings as commonly understood by those ordinarily skilled in the art. For a term explicitly defined herein, the meaning of the term is subject to the definition.

The term "effective amount" refers to an amount of a therapeutic agent that, when being administered alone or in combination with another therapeutic agent to cells, tissue or subjects, can effectively prevent or alleviate a disease condition or progression of the disease. The "effective amount" also refers to an amount of a compound that is sufficient to alleviate symptoms, e.g., to treat, cure, prevent or alleviate associated medical conditions, or to increase a rate at which such conditions are treated, cured, prevented or alleviated. When an active ingredient is administered alone to an individual, a therapeutically effective dose refers to the dose of the ingredient alone. When a certain combination is used, a therapeutically effective dose refers to a combined amount of active ingredients that produce a therapeutic effect, regardless of combined, sequential or simultaneous administration. The effective amount may be variably specified based on following factors, such as a formulation method, an administration method, and age, weight, sex, pathological condition, food, administration time, administration route, excretion rate and responsiveness of patients.

The term "tumor" refers to all neoplastic cell growth and proliferation, whether malignant or benign, and all pre-cancerous and cancerous cells and tissue. The terms "cancer" and "tumor" when referred to herein are not mutually exclusive.

The term "anti-tumor agent" refers to anti-tumor drug, which is a class of drugs for the treatment of tumor diseases, and includes chemotherapeutic drugs, biological agents, and the like.

The term "pharmaceutical preparation" or "pharmaceutical composition" refers to a preparation that is in a form permitting biological activity of active ingredients contained therein to be effective and that does not contain an additional component having unacceptable toxicity to a subject to be administered with the preparation. A vaccine adjuvant or a vaccine composition containing the vaccine adjuvant according to exemplary embodiments of the present invention may be formulated by a method known in the art, to enable rapid or sustained or delayed release when active ingredients are administered to mammals. These preparations may include powders, granules, tablets, emulsions, syrups, aerosols, soft or hard gelatin capsules, sterile injectable solutions and sterile powders.

The term "pharmaceutically acceptable" means that a composition, when being administrated to human beings, is physiologically acceptable and generally does not cause anaphylactic responses (such as gastrointestinal disorders and dizziness) or similar responses. Examples of delivery systems, excipients and diluting agents may include lactose, dextrose, sucrose, sorbitol, mannitol, xylitol, erythritol, maltitol, starch, acacia, sodium alginate, gelatin, calcium phosphate, calcium silicate, cellulose, methyl cellulose, polyvinyl pyrrolidone, water, methyl hydroxypropyl benzoate, talc, magnesium stearate, and mineral oil. In addition, fillers, anticoagulants, lubricants, humectants, fragrances, emulsifiers, preservatives, and the like may be further included.

The term "adjuvant" or "vaccine adjuvant" refers to a drug or immunologic preparation that is administered for improving immune responses to the vaccine. In addition to the vaccine adjuvants set forth in the present invention, the vaccine composition may further include conventionally used vaccine adjuvants. The conventionally used vaccine adjuvants may include aluminum hydroxide, aluminum phosphate, aluminum potassium sulfate, MF59, virosomes, AS04 [mixture of aluminum hydroxide and monophosphoryl lipid A (MPLA)], AS03 (mixture of DL-α-tocopherol, squalene and polysorbate 80), CpG, flagellin, poly (I:C), AS01, AS02, ISCOMs and so on.

The term "antigen" refers to a substance that induces immune responses. Thus, in the present invention, any substance exhibiting such activity of inducing immune responses may be used without limitation. The antigen may be peptide, protein, nucleic acid, sugar, pathogen, attenuated pathogen, inactivated pathogen, virus, virus-like particle (VLP), cell or cell fragment. The antigen may be selected from the group consisting of Japanese encephalitis virus antigen, Haemophilus influenzae type B (HIB) antigen, Middle East respiratory syndrome (MERS) virus antigen, Zika virus antigen, Pseudomonas aeruginosa antigen, pertussis antigen, Mycobacterium tuberculosis antigen, Bacillus anthracis antigen, hepatitis A virus (HAV) antigen, hepatitis B virus (HBV) antigen, hepatitis C virus (HCV) antigen, human immunodeficiency virus (HIV) antigen, herpes simplex virus (HSV) antigen, Neisseria meningitidis antigen, Corynebacterium diphtheriae antigen, Bordetella pertussis antigen, Clostridium tetani antigen, human papilloma virus (HPV) antigen, varicella virus antigen, *Enterococccus* antigen, *Staphylococcus aureus* antigen, *Klebsiella pneumoniae* antigen, *A. baumannii* antigen, *Enterobacter* antigen, *H. pylori* antigen, malaria antigen, dengue fever antigen, Orientia tsutsugamushi antigen, severe fever with thrombocytopenia syndrome bunyavirus (SFTS bunyavirus) antigen, severe acute respiratory syndrome coronavirus (SARS-CoV) antigen, influenza virus antigen, Ebola virus antigen and *Diplococcus pneumoniae* antigen.

### BRIEF DESCRIPTION OF DRAWINGS

FIG. 1 shows analysis of solution turbidity of antigen formulations of different formulas after 4 h oscillation (turbidity is detected by a turbidity meter, and unit of ordinates in the drawing is NTU).
FIG. 2 shows changes of polymer peak areas of antigen formulations of different formulas with different times of freeze-thawing, wherein the changes in peak area are detected by SEC-HPLC (ordinates represent percentage, for example, an ordinate change of 0.25 means 0.25% of peak area change); data of 0 times of freeze-thawing have slight change, because there will be certain detection deviation when protein formulated into different formulas is detected before freeze-thawing.
FIG. 3 shows analysis of serum antibody subtype IgG2a titers 14 days after second vaccination of C57BL/6 mice with vaccines in various test groups.
FIG. 4 shows cellular immunity analysis (CD4⁺ T-cell responses) 14 days after second vaccination of C57BL/6 mice with vaccines in various test groups.
FIG. 5 shows cellular immunity analysis (CD4⁺ T cell responses) 14 days after second vaccination of C57BL/6 mice with vaccines in various test groups.
FIG. 6 shows a path diagram of a preparation process of MA105 adjuvant system.
FIG. 7 shows a transmission electron micrograph of a recombinant herpes zoster vaccine.
FIG. 8 is a graph of particle size distribution of the recombinant herpes zoster vaccine.
FIG. 9 shows tumor growth curves of TC-1 tumor-bearing C57BL/6 mice after vaccination with MA105 adjuvant system + antigen.
FIG. 10 shows body weight change curves of TC-1 tumor-bearing C57BL/6 mice after vaccination with MA105 adjuvant system + antigen.
FIG. 11 shows IFN-y secretion levels in TC-1 tumor-bearing C57BL/6 mice after vaccination with MA105 adjuvant system + antigen.
FIG. 12 shows anti-tumor activity of MA105 adjuvant system alone (B16F10 tumor model, melanoma).
FIG. 13 shows anti-tumor activity of MA105 adjuvant system alone (MC38 tumor model, colorectal cancer).
FIG. 14 shows anti-tumor activity of MA105 adjuvant system alone (LLC tumor model, lung cancer).
FIG. 15 shows that MA105 adjuvant system can remarkably improve the therapeutic effect of anti-tumor antigen, wherein FIG. 15 (left) shows tumor volume after vaccination, and FIG. 15 (right) shows body weights of mice after vaccination.
FIG. 16 shows that mice generate remarkable immune protection after vaccination with MA105 adjuvant system + antigen.
FIG. 17 shows HPLC chromatogram of QS-21 sample after a third step of reverse phase fine purification (after four-step purification) during purification and preparation of QS-21.
FIG. 18 shows analysis of binding antibodies of recombinant herpes zoster vaccines of different processes.
FIG. 19 shows cellular immunity analysis (CD4⁺ T-cell responses) 14 days after second vaccination with recombinant herpes zoster vaccines of different processes.

### DETAILED DESCRIPTION OF EMBODIMENTS

### 1. Herpes Zoster Antigen

As a protein with the largest VZV surface content, gE protein is a transmembrane glycoprotein consisting of 623 amino acid residues, of which N-terminal 1-30 amino acids are a signal peptide, amino acids 31-538 are an extramembrane fragment, amino acids 539-559 are a transmembrane fragment, and amino acids 560-623 are an intramembrane region. The gE protein forms a dimer with glycoprotein gI on surface of the virus and plays an important role in infection of host cells by VZV. Both natural infection and VZV attenuated vaccine can induce the generation of gE-specific neutralizing antibodies and CD4⁺ T-cell immune responses, and thus gE is an important target of human immune responses. The most prominent site of antibody responses of the gE protein is located at its N-terminal 50-135 amino acid fragments, having at least one linear epitope that can be recognized by the neutralizing antibodies. The site where T cells recognize the gE protein is distributed over the entire 31-538 extramembrane fragments thereof. The gE protein transmembrane region does not contain a known antigen epitope or a T cell recognition site.

A gE protein sequence used in the antigen of the present invention is a common gE protein extramembrane region of human varicella zoster virus (Strain NH29_3 (ABH08489.1), Dumas (NP_040190.1), DE10-2480 (AKG56356.1) and VZV-VSD (AAG48520.1), the amino acid sequence is 31-538, without amino acid sequences in the transmembrane region and an intramembrane region.

Antigen production: each time 35-55 L of basal culture medium is fed, 1.5-3 L of cell suspension is inoculated, 8-10 L is supplemented, and a volume of a cell harvest solution is about 45-65 L. 5-10 g of gE antigen is finally obtained.

Antigen-expressing host cells: host cell CHO-K1 cells were purchased from Nanjing GenScript Biotech Corporation, with cell number CCL-61.

### 2. Tumor Antigen

Tumor antigen used in the present invention is a recombinant tumor antigen, which may be a tumor specific antigen/unique tumor antigen, tumor-associated antigen (TAA), and tumor neoantigen. Specifically, the tumor antigen includes, but is not limited to, an HPV antigen, a PSA antigen, a HER2 antigen, a CEA antigen, a VEGF antigen, an EGFR antigen, a MUC1 antigen, and the like. Preferably, the antigen is an HPV antigen having an amino acid sequence set forth in SEQ ID NO. 1, or an HPV antigen having an amino acid sequence set forth in SEQ ID NO. 2.
SEQ ID NO. 2: 
HQKRTAMFQDPQERPRKLPQLCTELQTTIHDIILECVYCKQQLLREVYDFAFRDLCIVYRDGNPY
AVGDKCLKFYSKVSEYRYYCYSLYGTTLEQQYNKPLCDLLIRGINCQKPLCPDEKQRHLDKKQRFHNI
RGRWTGRCMSCCRSSRTRRETQLgsgsgsgsgsgsgMHGDTPTLHEYMLDLQPETTDLYGYGQLHDSSEEE
DEIDGPAGQAEPDRAHYNIVTFCCKCDSTLRLCVQSTHVDIRTLEDLLMGTLGIVGPICSQKP
SEQ ID NO. 3:

ARFEDPTRRPYKLPDLCTELNTSLQDIEITCVYCKTVLELTEVFEFAFKDLFVVYRDSIPHAAGHK
CIDFYSRIRELRHYSDSVYGDTLEKLTNTGLYNLLIRGLRCQKPLNPAEKLRHLNEKRRFHNIAGHYRG
QCHSCCNRARQERLQRRRETQVgsgsgsgsgsgsgMHGPKATLQDIVLHLEPQNEIPVDLLGHGQLSDSEEE
NDEIDGVNHQHLPARRAEPQRHTMLCMCCKCEARIELVVESSADDLRAFQQLFLNTLSFVGPWCASQ
Q.

### 3. Other Raw Materials

Commercially available products conforming to drug standard, including
DOPC: dioleoyl-phosphatidylcholine;
DOTAP: 1,2-dioleoyl-3-trimethyl ammonium chloride propane;
cholesterol; and
Poly (I:C): (polyinosinic:polycytidylic acid).

### Example 1. Screening of Auxiliary Materials for Antigen Stability

In order to ensure long-term stable storage of the gE protein at -70°C, we have screened a variety of auxiliary materials in earlier stage, studied their protective effects on the protein under a 50°C heating treatment and under a room-temperature oscillation condition, and evaluated changes and differences in OD 350 value, turbidity, IEC-HPLC-detected main peak area and SDS-PAGE before and after sample treatment.
1. Experiments found that Tween-80 can effectively reduce aggregation of antigen protein in preparations under an oscillation condition, and glycerol, histidine, sorbitol and sucrose can effectively reduce protein degradation under a high temperature condition.
2. Since glycerol is generally not used for preserving a primary liquid (mother solution), sorbitol and histidine are selected as a buffer system, and meanwhile it is considered that sucrose is the first choice of auxiliary material of preparation in most recombinant biological products, 10% sucrose is selected as a thermal stability protective agent. Finally, 10% sucrose + 10 mM histidine + 0.025% polysorbate 80 (Tween-80), pH6.5, were chosen as a formula of primary liquid.

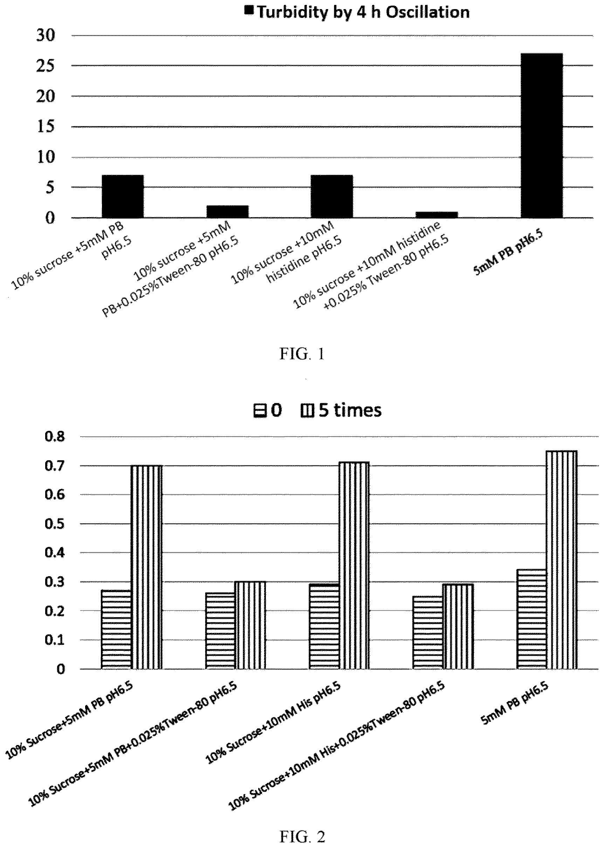

Many formulas were screened, and solution turbidity of different formulas was investigated, with results being shown in following **Table 1** and **FIG. 1**. Antigen polymerization of solvent systems of different formulas after repeated freeze-thawing was further investigated, and results are shown in **Table 1** and **FIG. 2****.**

**Table 1. Screening of Antigen Solution Formulas**

| Buffer System Formula | Turbidity after 4 h Oscillation | Peak Area Change Rate Detected after 0 Times of Freeze Thawing | Peak Area Change Rate Detected after 5 Times of Freeze Thawing |
|---|---|---|---|
| 10% sucrose +5mM PB pH6.5 | 7 | 0.27 | 0.7 |
| 10% sucrose +5mM PB+0.025%Tween-80 pH6.5 | 2 | 0.26 | 0.3 |
| 10% sucrose +10mM histidine pH6.5 | 7 | 0.29 | 0.71 |
| **10%** sucrose **+10mM** histidine **+0.025%Tween-80 pH6.5** | **1** | **0.25** | **0.29** |
| 5mM PB pH6.5 | 27 | 0.34 | 0.75 |

Y202009-15 batch samples were prepared according to the above formulas, and stability test was carried out. Specific stability test parameters and steps are as follows:
Long term condition (-70°C), sampling points: 0 days, 3 months, 6 months; and
Accelerated condition (2-8°C), sampling points: 0 days, 3 days, 7 days, 14 days.

Detection results show that samples are stable, and qualified in appearance, protein content, antigen activity, electrophoretic purity check and HPLC purity check, and sterility test. Isoelectric point, peptide map and N-terminal sequencing results show no change in antigen protein properties. It indicates that the recombinant herpes zoster vaccine primary liquid can at least tolerate 4 rounds of freeze-thawing.

### Example 2. Screening of Formulations of Herpes Zoster Vaccine Preparation

The herpes zoster vaccine preparation of the present invention is a suspension consisting of a recombinant varicella zoster virus glycoprotein E (gE antigen) and an MA105 adjuvant system. The MA105 adjuvant system is a liposome preparation containing two immunity-enhancing ingredients, that is, QS-21 and poly (I:C). We determined the formulation of the MA105 adjuvant system and the content of effective antigen by screening formulations, and determined a formulation process of a semi-finished product through development of production process.

The adjuvant system contains two immunopotentiating agents (QS-21 and Poly I:C). In order to reduce the toxicity of QS-21 and poly (I:C) and improve immunogenicity, we used a mixture of the cationic and neutral liposomes to adsorb QS-21 and poly (I:C).

Signal transduction of poly (I:C) relies primarily on Toll-like receptor 3 (TLR3) and melanoma differentiation-associated gene 5 (MDA-5), and can strongly drive cellular immunity and effective type I interferon responses, and can induce strong antigen-specific CD4⁺ T and CD8⁺ T-cell responses through type I interferon (IFN-α/β) signal transduction and antigen cross presentation. As the poly (I:C) receptor is located on intracellular membranes, in order to target intramuscular poly (I:C) to lymph nodes and reduce side effects caused by its diffusion into blood, we used cationic liposomes (consisting of DOTAP, DOPC and cholesterol) as a delivery system to adsorb poly (I:C) by electrostatic interaction.

Studies found that **large mass of floc or sediment appeared after the cationic liposome alone adsorbed poly (I:C). In order to maintain stability of physical properties of products, the mixed liposomes containing the cationic liposomes and the neutral liposomes (consisting of DOTAP and cholesterol) were chosen in the present invention to adsorb poly (I:C)**. Poly (I:C) adsorbed by the intramuscular administered mixed liposomes could be passively or actively delivered to antigen presenting cells (APCs), to limit its interaction with non-phagocytic cells, and regulate systemic secretion of cytokines generated thereby. Toxicology studies of the present product have proved that adsorption of poly (I:C) onto the mixed liposomes can effectively improve its safety.

Free QS-21 binds to cholesterol on cell membranes, to form an irreversible complex, which when binding to red blood cells causes hemolysis. When the QS-21 is adsorbed to cholesterol in the mixed liposomes, its binding to the cell membrane is prevented, thus reducing the hemolytic effect. Experiments prove that the addition of cholesterol in the adjuvant system of the present invention can adsorb at least 3 times the dose of QS-21 of the finished product without causing hemolysis, which indicates that the cholesterol content is in excess, and can reduce potential risks caused by the hemolytic activity of QS-21.

Vaccines formulated using a single immunopotentiating agent (poly (I:C) or QS-21) and the mixed liposomes in combination are all capable of inducing a high level of antibodies and a high frequency of CD4+ T cells. Vaccines formulated by a combination of the two immunopotentiating agents and the mixed liposomes induced the generation of an even higher level of binding antibodies and a higher frequency of CD4+ T cells, which indicates that poly (I:C) and QS-21 have a synergistic effect on immune responses. Therefore, the combined use of the two immunopotentiating agents further contributes to improving the immunogenicity of the present product and the number of CD4⁺ T cells, as described in detail in Example 4.

Polysorbate 80 (Tween-80) was chosen for avoiding flocculent aggregates in the finished product. Sodium chloride was used for regulating osmotic pressure of the vaccine and maintaining systemic osmotic pressure (210-350 mOsmol/kg). Sucrose is used to maintain the stability of the liposomes.

Specific information on active ingredients and auxiliary materials in each 1 ml of vaccine preparation is shown in Table 2.

**Table 2. List of Contents of Main Ingredients in Herpes Zoster Vaccine Preparation**

| Ingredient Name | Formula 1 | Formula 2 | Function |
|---|---|---|---|
| | Content (µg) | Content (µg) | |
| gE | 100 | 100 | Antigen |
| DOTAP | 140 | 140 | Auxiliary material (adjuvant) |
| DOPC | 1260 | 2000 | Auxiliary material (adjuvant) |
| Cholesterol | 350 | 500 | Auxiliary material (adjuvant) |
| QS-21 | 100 | 100 | Auxiliary material (adjuvant) |
| Poly I:C | 800 | 800 | Auxiliary material (adjuvant) |
| Polysorbate 80 | 500 | 500 | Auxiliary material (stablize liposome) |
| Histidine | 914 | Not higher than 233 | Auxiliary material (stablize pH value) |
| Sodium dihydrogen phosphate | - | 715 | Auxiliary material (stablize pH value) |
| Sucrose | 54000 | 54000 | Auxiliary material (stablize liposome) Auxiliary material |
| Sodium chloride | 2720 | 4200 | (regulate osmotic pressure) |

| | | | |
|---|---|---|---|
| DOPC: dioleoyl-phosphatidylcholine; DOTAP: 1,2-dioleoyl-3-trimethyl ammonium chloride propane; cholesterol; QS-21: Quillaja saponaria saponin 21; and Poly (I:C): polyinosinic: polycytidylic acid. | | | |

### Example 3. Preparation and Testing of Herpes Zoster Vaccine

Formulation steps of the vaccine preparation are as follows.

### 3.1. Preparation of cationic liposomes:

The cationic liposomes are formulated from 1,2-dioleoyl-3-trimethyl ammonium chloride propane (DOTAP), dioleoyl phosphatidylcholine (DOPC) and cholesterol according to a mass ratio of 2:2:1 by an ethanol injection method. Specific preparation steps are as follows:
(1) dissolving the three lipid ingredients in ethanol and shearing the resultant on line with water phase for 1-3 times;
(2) buffer replacement by ultrafiltration using a sucrose-containing buffer solution, concentration 2-4 times and washing-filtering 6-8 times;
(3) extruding 200 nm liposomes by a filter membrane with a pore size of 200 nm, performing multiple rounds of extrusion, and controlling particle size homogeneity;
(4) extruding 100 nm liposomes by a filter membrane with a pore size of 100 nm, performing multiple rounds of extrusion, and controlling particle size homogeneity; and
(5) sterilizing by filtration.

Physical and chemical parameters of the prepared cationic liposomes are as follows:
average particle size (D50): 60-120 nm;
solution pH value: 4.8-7.0; and
Zeta potential: 40-72 mV (pH 6.5).

The cationic liposomes are stored at 2-8°C. After long-term stability investigation for 12 months, the cationic liposomes are stable in morphology and ingredients.

### 3.2. Preparation of neutral liposomes:

The neutral liposomes are a lipid bilayer globule consisting of dioleoyl phosphatidylcholine (DOPC) and cholesterol, wherein a mass ratio of DOPC to cholesterol is 4:1. Specific preparation steps are as follows:
(1) dissolving two lipid ingredients in ethanol and shearing the resultant on line with water phase for 1-3 times;
(2) extruding 200 nm liposome by a filter membrane with a pore size of 200 nm, performing multiple rounds of extrusion, and controlling particle size homogeneity;
(3) extruding 100 nm liposome by a filter membrane with a pore size of 100 nm, performing multiple rounds of extrusion, and controlling particle size homogeneity;
(4) ultrafiltration replacement using a sucrose-containing buffer solution, concentration 3-6 times and washing-filtering 6-8 times; and
(5) sterilizing by filtration.

Physical and chemical parameters of the prepared neutral liposomes are as follows:
average particle size (D50): 80-140 nm; and
solution pH value: 5.5-7.0, preferably 6.4-6.6.

The neutral liposomes are stored at 2-8 °C. After long-term stability investigation for 12 months, the cationic liposomes are stable in morphology and ingredients.

### 3.3. Formulation of vaccine

The vaccine is formulated using the ingredients with final contents according to proportions described in **Table 2** of Example 2.

The cationic liposomes, neutral liposomes and various auxiliary materials are added according to amounts calculated according to concentrations in Table 2, then QS-21, poly (I:C) and gE antigen solution are added respectively, and pH is adjusted to 6.0-7.0.

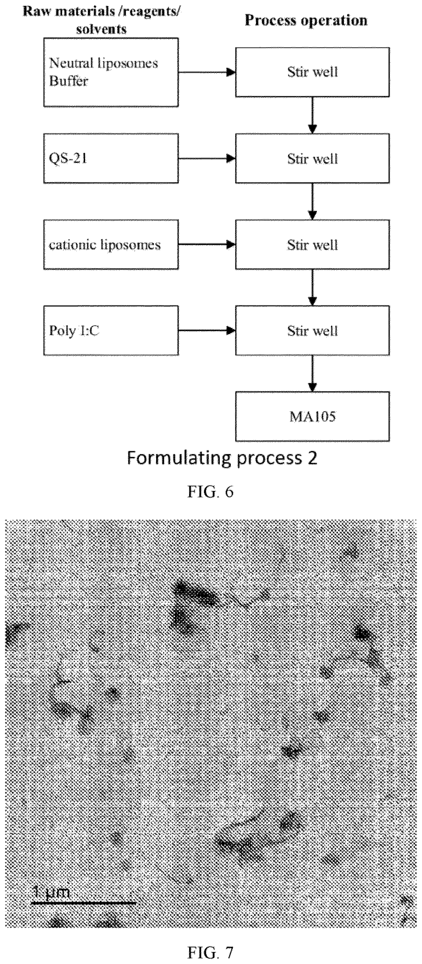

A complete preparation process for the vaccine is shown in **FIG. 6****.**

### 3.4. Quality control of vaccine preparations

Multiple batches of vaccines were prepared according to the above method, specifically as shown in **Table 3** below.

**Table 3. Records of Vaccine Preparation Production Batches**

| Pilot Batch | 50L Fermentation | | |
|---|---|---|---|
| Batch No. | C202007-01 | C202009-03 | C202009-04 |
| Lot Size | 2172 vials | 987 vials | 1009 vials |
| Production Date | 2020.07.31 | 2020.09.29 | 2020.09.29 |

Products were monitored for quality, and contents of main ingredients were assessed. Results are as shown in following **Table 4**

**Table 4. Quality Control Results of Vaccine Preparations**

| Verification Item | Applicable Detection Criteria | Pilot batch | | |
|---|---|---|---|---|
| | | C202007-01 | C202009-03 | C202009-04 |
| Physical Inspection | | | | |
| Apperance | | Milk white suspension | Milk white suspension | Milk white suspension |
| Osmotic Pressure Molar Concentration mOsmol/kg | Measure according to pharmacopeia (general chapter 0632) | 273 | 260 | 260 |

| Chemical Verification | | | | |
|---|---|---|---|---|
| pH Value | Measure according to pharmacopeia (general chapter 0631) | 6.78 | 6.53 | 6.53 |
| Cholesterol Content mg/ml | Measure according to pharmacopeia (general chapter 0512) | 0.34 | 0.38 | 0.35 |
| DOPC Content mg/ml | Measure according to pharmacopeia (general chapter 0512) | 1.20 | 1.47 | 1.30 |
| DOTAP Content mg/ml | Measure according to pharmacopeia (general chapter 0512) | 0.15 | 0.16 | 0.15 |
| QS-21 Content µg/ml | Measure according to pharmacopeia (general chapter 0512) | 81 | 95 | 96 |
| Poly I:C Content mg/ml | Measure according to pharmacopeia (general chapter 0512) | 0.83 | 0.77 | 0.76 |
| Polysorbate 80 Content mg/ml | Measure according to pharmacopeia (general chapter 0512) | 0.47 | 0.48 | 0.47 |

### Example 4. Control Testing of Herpes Zoster Vaccine Preparations and Comparison of Immunogenicity of Herpes Zoster Vaccines of Different Formulations

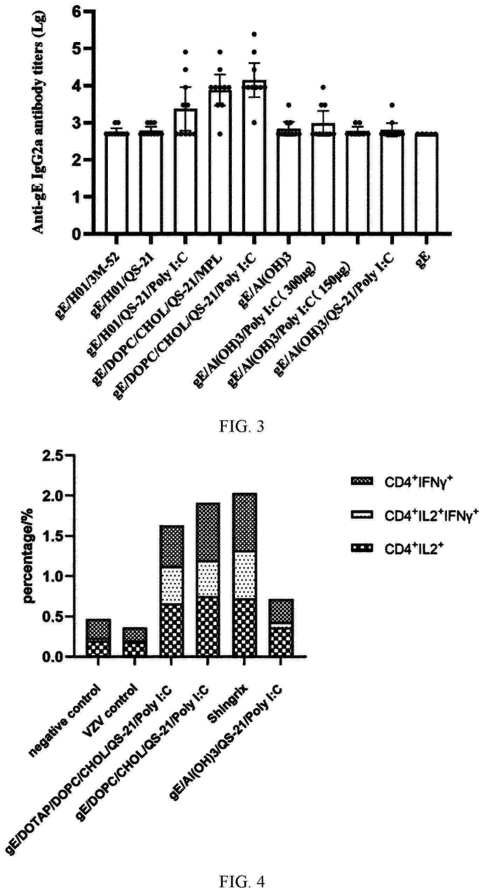

The vaccine of the formulation of the present invention, aluminum hydroxide adjuvant formula (gE/nano aluminum hydroxide adjuvant/QS-21/Poly I:C) as Control Group 1; Shingrix^{®} formula as Control Group 2, and neutral liposome formulation (gE/neutral liposome/QS-21/Poly I:C) as Control Group 3 were chosen to assess vaccination effects in C57BL/6 mice, and the results show the following:
(1) The mice vaccinated with gE antigen formulated with four different formulations all generated a high titer of gE binding antibodies. The formulation with mixed liposomes of the present invention was superior to other formulations. Detection results are shown in detail in **FIG. 3**, wherein:
   concentrations of gE antigen in all vaccine formulations were consistent (100 µg/ml);
   MPL, 3M-52 (TLR7/8 agonist), aluminum hydroxide, and Poly I:C were immunologic adjuvants;
   the gE group was a pure antigen solution free of liposomal preparation;
   the nanoemulsion group was an oil-in-water formulation;
   the formulation method for the neutral liposomes is described in Example 3 section 3.2, and the amount of the neutral liposome used in the reference preparation of the neutral liposome is consistent with that in the nanoemulsion group; and
   buffer solutions, solvents, QS-21, and poly (I:C) (excluding poly (I:C) high and low dose groups) used in all groups remained consistent, and amounts of ingredients are shown in **Table 2.**
(2) The mixed liposomal formulation and the neutral liposomal formulation of the present invention and the Shingrix^{®} formulation could all induce and stimulate the generation of CD4⁺ T-cell responses, without significant difference between the groups, and they were all superior to the aluminum hydroxide formulation. Results are shown in detail in **FIG. 4**, wherein
   concentrations of gE antigen in all reference preparation groups were consistent (100 µg/ml);
   blank control was free of antigen, but the vaccine preparation had a complete formulation;
   virus control contained antigen alone, free of the mixed liposomes, poly (I:C) and QS-21;
   Shingrix is a commercially available vaccine product group;
   the mixed liposomal group was a formulation of mixed cationic liposomes and neutral liposomes as set forth in section 3.3 of Example 3;
   the formulation of the neutral liposomes was as described in Example 3 section 3.2, and the amount of the neutral liposomes used in the reference preparation of the neutral liposomes was consistent with that in the nanoemulsion group; and
   buffer solutions, solvents, QS-21, and poly (I:C) used in all groups remained consistent, and amounts of ingredients are shown in **Table 2.**

In order to study the immune effects of different formulations, we carried out tests shown in the following table, wherein two formulation are shown in **Table 2,** two formulation methods for the MA105 adjuvant system are shown in **FIG. 6**, and sample information is shown in the following table.

| Sample | Formula | Formulating Process |
|---|---|---|
| Sample 1 | Formula 1 | Formulating Process 1 |
| Sample 2 | Formula 1 | Formulating Process 2 |
| Sample 3 | Formula 2 | Formulating Process 1 |
| Sample 4 | Formula 2 | Formulating Process 2 |

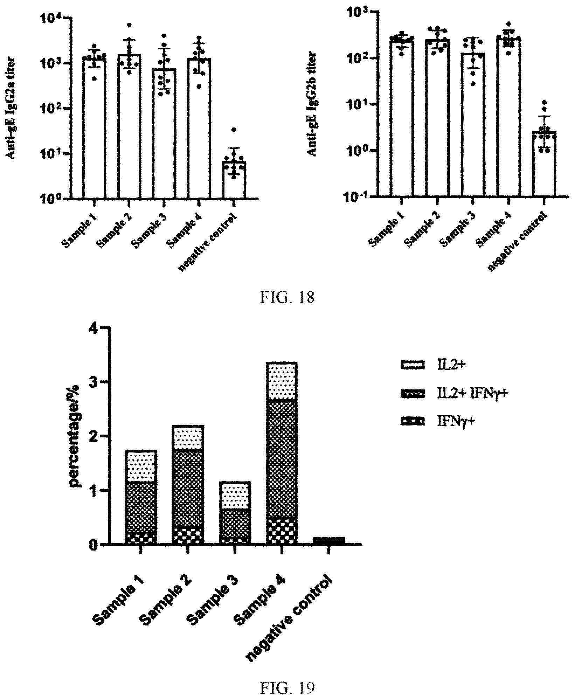

Results show that different formulations and formulation methods are able to induce the generation of a high level of binding antibodies, without significant difference between the groups (**FIG. 18**); but sample 4 induced the generation of a higher frequency of (IL-2/IFN-γ) CD4⁺ T cells (**FIG. 19**).

### Example 5. Comparison of Properties of Different Vaccine Preparations

### 5.1. Synergistic effect of immunopotentiating agents

To study influence of a single immunopotentiating agent (poly (I:C) or QS-21) on vaccine immunogenicity, and whether poly (I:C) and QS-21 in the formula have a synergistic effect on vaccine immunogenicity, on the basis of the vaccine formulations determined in Example 2 and preparation route determined in Example 3, a vaccine formulation of a single immunopotentiating agent (poly (I:C) or QS-21) and the mixed liposomes was compared with a vaccine formulation containing both poly (I:C) and QS-21 and the mixed liposomes for immunogenicity.

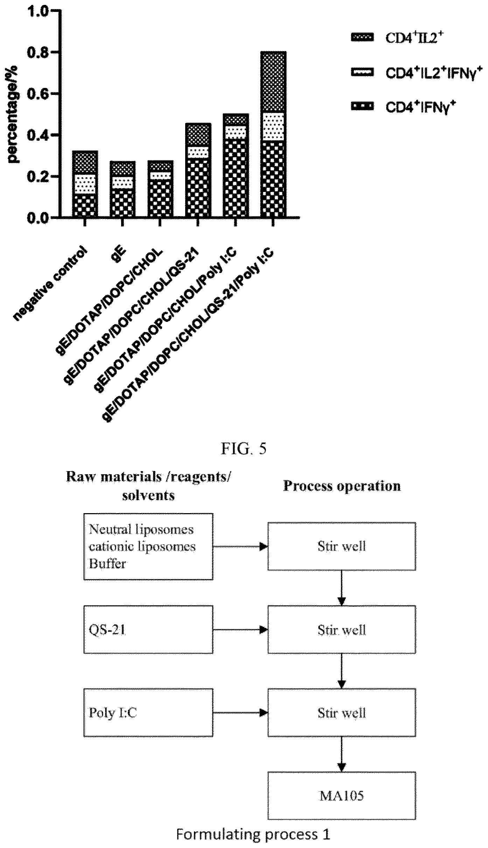

The vaccines formulated by a combination of the immunopotentiating agent and the liposomes could all induce the generation of a high level of binding antibodies, and induce the generation of a high frequency of (IL-2/IFN-γ) CD4⁺ T cells, but the gE/composite adjuvant (poly (I:C) and QS-21) candidate vaccine induced the generation of a higher level of binding antibodies and a higher frequency of (IL-2/IFN-γ) CD4⁺ T cells, which indicates that poly (I:C) and QS-21 have a synergistic effect on immune responses. Results are shown in detail in **FIG. 5**, wherein
preparation methods for preparations of various reference groups are described in Example 3, and differences between specific preparation ingredients are as follows:
blank control group: free of antigen, but the vaccine preparation had a complete formulation;
gE buffer solution group: free of mixed liposomal preparation, poly (I:C) and QS-21;
gE mixed liposome group: mixed liposome preparation, free of poly (I:C) and QS-21;
gE mixed liposome/QS-21 group: mixed liposome preparation, free of poly (I:C);
gE mixed liposome/poly (I:C) group: mixed liposome preparation, free of QS-21; and
gE mixed adjuvant group: complete preparation prepared in Example 3.

### 5.2. Optimization of poly (I:C) and QS-21 doses

### 5.2.1 Optimization of poly (I:C) dose

Immunogenicity of gE/ adjuvant candidate vaccines formulated with different contents of poly (I:C) (400 µg/ml, 800 µg/ml, 1600 µg/ml) was compared to determine the optimal content of Poly I:C in the vaccine formulation.

The gE/composite adjuvant candidate vaccines containing different poly (I:C) doses could all generate a high level of binding antibodies, without significant difference between the groups, and could all induce the generation of a high frequency of (II,-2/IFN-y) CD4⁺ T cells. Considering that the stability and transmission efficiency of poly (I:C) can be improved using the mixed liposomes, the content of poly (I:C) in the vaccine was provisionally set to be 800 µg/ml.

### 5.2.2. Optimization of QS-21 dose

Immunogenicity of gE/composite adjuvant candidate vaccines formulated with different contents of QS-21 (100 µg/dose, 50 µg/dose, 25 µg/dose, 12.5 µg/dose) was compared to determine the content of QS-21 in the vaccine formulation.

The gE/composite candidate vaccines containing different QS-21 doses, upon vaccination, all stimulated the generation of a high level of binding antibodies. Each group in the test induced (IL-2/IFN-γ) CD4⁺ T-cell responses. With reference to the Shingrix^{®} formulation, QS-21 concentration in the present product was provisionally set to be 50 µg/dose (100 µg/ml).

### 5.3 Antigen content study

The present vaccine is a liquid preparation containing a novel adjuvant. According to requirements of "Preclinical Study Technology Guideline for Prophylactic Vaccine", four groups of gE vaccines formulated with different contents of gE antigen (100 µg/dose, 50 µg/dose, 25 µg/dose, 5 µg/dose, 0 µg/dose, 0.5 ml per dose) and pure adjuvant control were compared, to study the effect of the content of gE antigen on vaccine immunogenicity.

Within the research range of antigen contents of the present test, the vaccines formulated with different contents of gE antigen could all induce the generation of a high level of binding antibodies and a high frequency of (IL-2/IFN-y) CD4⁺ T cells. Humoral and cellular immune response results show that the 50 µg/dose and higher contents of the gE antigens can induce strong immune responses. The antigen was finally determined to be 50 µg/dose (concentration being 100 µg/ml).

### Example 6. Preparation of Anti-tumor Vaccine Preparations

Cationic liposomes and neutral liposomes in MA105 adjuvant were prepared with reference to contents in Example 3, sections 3.1-3.2

### 6.1. Formulation of vaccine preparation

The vaccine was formulated according to the final contents of various ingredients of the vaccine preparation in Example 2 according to the proportions in **Table 5**:

**Table 5. List of Contents of Various Ingredients in Anti-tumor Vaccine Preparation (per dose)**

| Ingredient Name | Content (µg) | Function |
|---|---|---|
| E6 Antigen | 250 | Antigen |
| DOTAP | 70 | Auxiliary material (adjuvant) |
| DOPC | 630 | Auxiliary material (adjuvant) |
| Cholesterol | 175 | Auxiliary material (adjuvant) |
| QS-21 | 50 | Auxiliary material (adjuvant) |
| Poly I:C | 400 | Auxiliary material (adjuvant) |
| Polysorbate 80 | 250 | Auxiliary material (stablize liposome) |
| Histidine | 457 | Auxiliary material (stablize pH value) |
| Sucrose | 27000 | Auxiliary material (stablize liposome) Auxiliary material |
| Sodium chloride | 1400 | (regulate osmotic pressure) |

The antigen is an HPV E6 antigen having an amino acid sequence set forth in SEQ ID NO. 2.

### Example 7. Activity Detection of Anti-tumor Vaccine Preparations (TC-1 tumor model)

### 7.1. Experimental Scheme

TC-1 tumor-bearing mice (C57BL/6) were randomly grouped, 7 mice in each group, vaccinated in groups, and subsequently observed and detected.

Vaccination procedure: 7 days after tumor inoculation (subscapular inoculation of tumor mass cut from a previous generation of TC-1 tumor-bearing mice in a lower part at one side), when a short diameter grew to about 9 mm, primary vaccination was performed, by subcutaneous muscle injection, 200 µL per mouse (formulations of the vaccine preparations are shown in Table 6, 200 µL of preparation in each group was administered per vaccination). A second and third vaccination were performed at 14 d and 21 d respectively after tumor transplantation.

### Detection points:

(1) TC-1 tumor-bearing mice were observed every 3 days after vaccination, and long and short diameters of tumors, body weights of the mice, and general conditions of the mice were recorded.
(2) Splenic lymphocytes of TC-1 tumor-bearing mice were isolated and IFN-y secretion was detected by ELISPOT.

**Table 6. Formulations of Anti-tumor Vaccine Preparations**

| Group | Main Branch | Content of Main Ingredient of Antigen and Adjuvant |
|---|---|---|
| NC (negative control) | Buffer | 0.3M Arginine and 10mM PB |
| Antigen (antigen group) | Antigen (701) | Antigen: 250µg/ml |
| A (control adjuvant A) | Antigen + Adjuvant A | Antigen: 250µg/ml |
| | | Squalene: 10.69mg/ml |
| | | Vitamin E: 11.86mg/ml |
| | | Tween 80: 4.86mg/ml |
| | | Toll7/8: 5pg/ml |
| D (MA105 adjuvant) | Antigen +MA105 | Antigen: 250µg/ml |
| | | QS-21: 50µg/ml |
| | | Poly I:C: 400µg/ml |
| | | DOTAP: 70µg/ml |
| | | DOPC: 630µg/ml |
| | | Cholesterol: 175µg/ml |

The antigen used was an HPV antigen having an amino acid sequence set forth in SEQ ID NO. 2.

### 7.2. Experiment Results

### (1) Tumor growth curves of TC-1 tumor-bearing mice

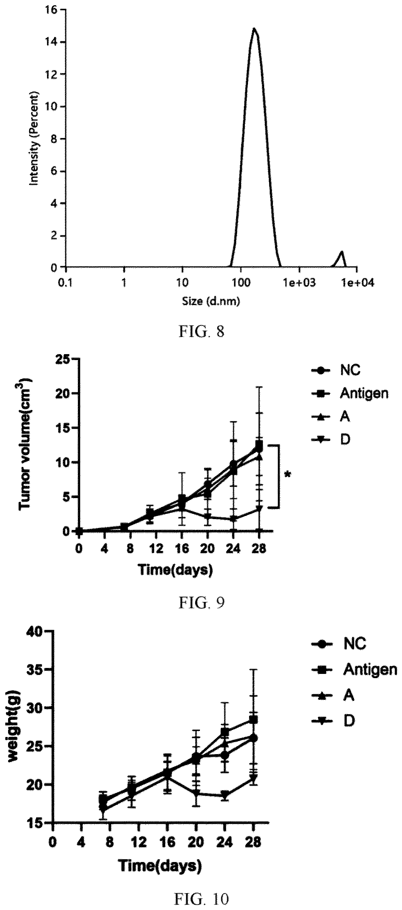

During the primary vaccination, mean tumor volumes of various groups were 0.59 cm³, 0.62 cm³, 0.68 cm³, 0.77 cm³, 0.68 cm³, without statistical difference, and the tumor growth curves of the TC-1 tumor-bearing mice are shown in **FIG. 9**. Results show that compared with NC group, the D treatment group exhibits significant tumor suppression, with tumor volume suppression rates of 82.16% (24 d) and 73.30% (28 d).

In the graph, tumor volume calculation equation is: V = 4/3 * π * long diameter * short diameter²

Tumor suppression rate calculation equation is: suppression rate = (mean volume of transplanted tumor in NC group - mean volume of transplanted tumor in experimental group) / mean volume of transplanted tumor in NC group * 100%.

### (2) Body weight change curves of TC-1 tumor-bearing mice

The body weight curves of TC-1 tumor-bearing mice are shown in **FIG. 10**. Results show that after the administration of therapies to various groups was completed, the body weights of the mice were not significantly reduced (a reason for the increased body weights is tumor growth), which indicates that the vaccines in various groups do not have overt toxicity.

### (3) IFN-y secretion levels in TC-1 tumor-bearing mice after vaccination three times

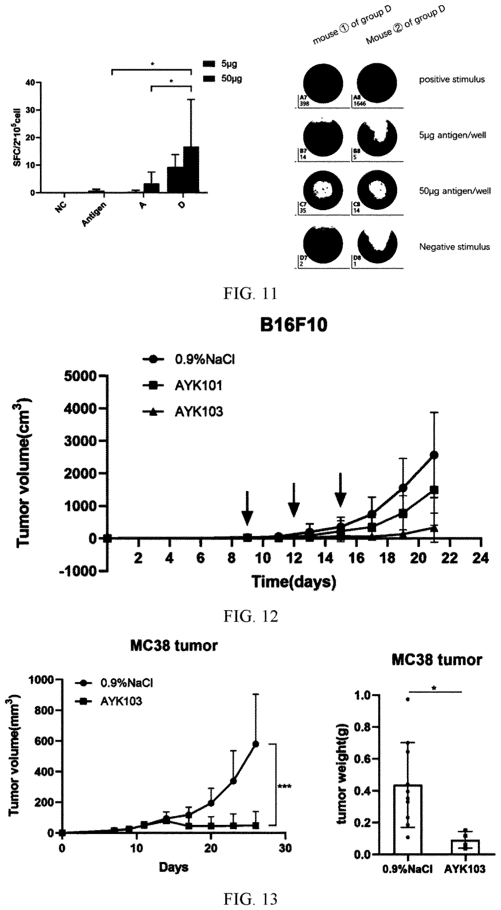

10 days after vaccination three times, three mice were randomly selected from each group to undergo splenic lymphocyte isolation and culture, stimulated with 5 ug and 50 ug of antigens respectively. IFN-γ secretion levels in TC-1 tumor-bearing mice were detected using Dayou ELISPOT-IFN-γ kits. Results are shown in **FIG. 11**. Compared with the NC group, the D group exhibits significant activation of IFN-γ secreting cells.

The above results indicate that the MA105 adjuvant system can enhance the immune effect of a tumor antigen and improve the therapeutic activity of a tumor vaccine.

### Example 8. Detection of Anti-tumor Activity of Antigen-free MA105 Adjuvant System (B16F10 tumor model)

### Experimental Scheme

B16F10 tumor-bearing mice (C57BL/6) were randomly grouped, 8 mice per group, vaccinated, and subsequently observed and monitored.

Vaccination procedure: after tumor inoculation (each mouse was inoculated with 50,000 B16F10 tumor cells), the mice were vaccinated on days 9, 12 and 15 by an administration method of intratumoral injection, and only antigen-free adjuvant was administered as an immunotherapy, 100 µL per mouse each time (formulations are shown in Table 7). On day 18, mice without tumor were inoculated with tumor cells for the second time (inoculated with 50,000 B16F10 tumor cells)

Detection points: after primary treatment, the mice were observed every 2 days, and long and short diameters of tumors, body weights of the mice, and general condition of the mice were recorded.

**Table 7 Formulas of Antigen-free Adjuvant**

| Group | Content of Main Ingredient |
|---|---|
| Normal Saline (negative control) | 0.3M Arginine and 10mM PB |
| AYK101 (control adjuvant A) | Squalene: 10.69mg/ml |
| | Vitamin E: 11.86mg/ml |
| | Tween 80: 4.86mg/ml |
| | Toll7/8: 5µg/ml |
| AYK103 (MA105 Adjuvant) | QS-21: 50µg/ml |
| | Poly I:C: 400µg/ml |
| | DOTAP: 70µg/ml |
| | DOPC: 630µg/ml |
| | Cholesterol: 175µg/ml |

Tumor growth curves of B16F10 tumor-bearing mice are shown in **FIG. 12**.

Results show that compared with other groups, the AYK103 (MA105 adjuvant system) administration group exhibited significant tumor suppression.

### Example 9. Detection of Anti-tumor Activity of Antigen-free MA105 Adjuvant System (homograft tumor model of colorectal cancer MC38 cell line)

### Experimental scheme

MC38 tumor-bearing mice (C57BL/6) were randomly grouped, 10 mice per group, vaccinated, and subsequently observed and monitored.

Vaccination procedure: after tumor inoculation (each mouse was inoculated with 1×10⁵ MC38 tumor cells), the mice were vaccinated on days 11, 14, and 17 (intratumoral injection), and only antigen-free adjuvant was administered for immunotherapy, 100 µL per mouse each time (MA105 formulation is shown in preceding **Table 7**).

Detection points: after primary treatment, the mice were observed every 3 days, and long and short diameters of tumors, body weights of the mice, and general condition of the mice were recorded. At the end of the experiment, the mice were sacrificed, and tumor tissue was removed and weighed.

Tumor growth curves of MC38 tumor-bearing mice and analysis of tumor weights of various groups at the end of the experiment are shown in **FIG. 13**.

The results show that compared with the normal saline control group, the MA105 adjuvant administration group exhibited significant tumor suppression.

### Example 10. Detection of Anti-tumor Activity of Antigen-free MA105 Adjuvant System (homograft tumor model of lung cancer LLC cell)

### Experimental scheme

LLC tumor-bearing mice (C57BL/6) were randomly grouped, 10 mice per group, vaccinated, and subsequently observed and monitored.

Vaccination procedure: after tumor inoculation (each mouse was inoculated with 6×10⁵ LLC tumor cells), the mice were vaccinated on days 11, 14, and 17 (intratumoral injection), and only antigen-free adjuvant was administered for immunotherapy, 100 µL per mouse each time (MA105/AYK103 formulations are as shown in preceding **Table 7**).

Detection points: after primary treatment, the mice were observed every 3 days, and body weights of the mice and general condition of the mice were recorded. At the end of the experiment, the mice were sacrificed, and tumor tissue was removed and weighed.

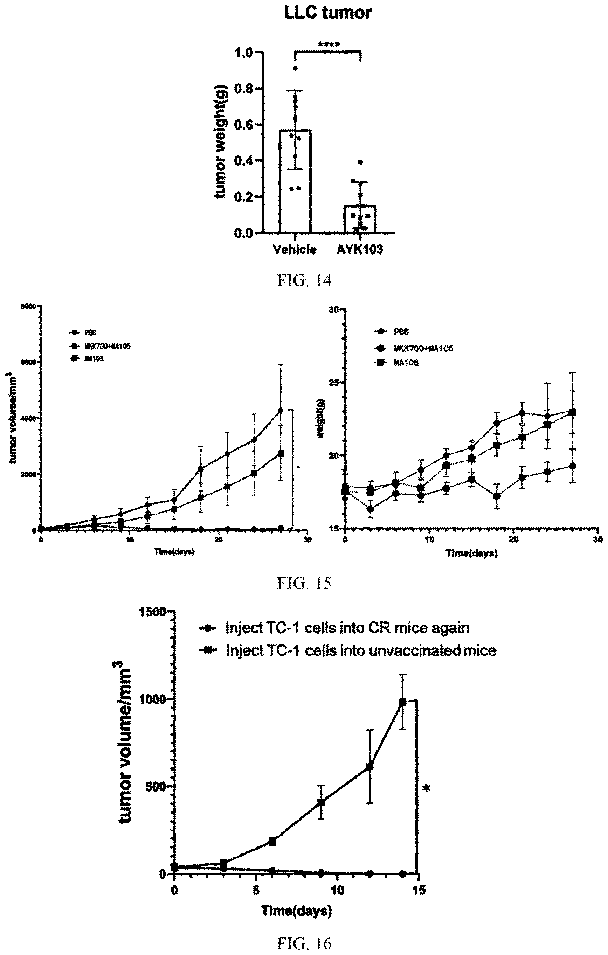

During the study, LLC tumors grew flat, and therefore tumor volume growth curves could not be measured. Analysis of tumor weights of various groups at the end of the experiment is shown in **FIG. 14**.

Results show that compared with the normal saline control group, the MA105 adjuvant administration group exhibits significant tumor suppression.

### Example 11. Immunoprotective Efficacy Assay of Vaccine Preparations (antigen + MA105 adjuvant) (TC-1 tumor model)

### Experimental scheme

(1) Tumor-bearing mouse models were constructed according to the method of Example 2. TC-1 tumor-bearing mice (C57BL/6) were randomly grouped, 5 mice per group. When a short diameter of transplanted tumor grew to about 5 mm, primary vaccination was performed (7 d), and second vaccination and third vaccination were performed respectively on day 14 and day 21.
(2) The left and right hind limb of each mouse was intramuscularly injected with 200 µL of vaccines of the following groups:
   1) NC Group (PBS 200 µL);
   2) MKK700 + MA105 group: MKK700 antigen was an HPV antigen having an amino acid sequence set forth in SEQ ID NO. 1 + MA105 group (PBS buffer solution 20 µL + MKK700 antigen 80 µL + MA105 adjuvant 100 µL, and concentrations and doses of antigen and adjuvant are the same as in Example 2); and
   3) MA105 group (PBS buffer solution 100 µL + MA105 adjuvant 100 µL).

Formulations of the antigen solution and MA105 adjuvant are as shown in **Tables 6 and 7**.

After administration, tumor growth curves of the mice are shown in **FIG. 15**, in which in the mice in the antigen + MA105 adjuvant administration group, 60% of the mice (3/5) achieved CR (complete remission) and 40% (2/5) achieved PR (partial remission).

Subsequently, TC-1 tumor mass was implanted again into three mice having achieved complete remission after the treatment in a different location (lower part of contralateral scapulae). The results show that the antigen + MA105 adjuvant treatment has a sustained immunoprotective effect, and the mice having achieved complete remission generate strong immunity against the reimplanted TC-1 tumor, such that the tumor is unable to grow (**FIG. 16**).

### Example 12. Purification and Preparation of QS-21

### Main raw materials, equipment and reagents

Crude saponin (derived from Quillaja saponaria dry extract) was purchased from Desert King (Name: VET-SAP, CAS No: 8047-15-2)

Upon high-performance liquid chromatography analysis of the raw materials, QS-21 content was not lower than 8%, moisture content was not higher than 10%, and ash content was not higher than 3%, and heavy metal content and pesticide residue met the drug standard.

UniPSN 30-300 chromatographic medium was from Suzhou Nanomicro Technology Co., Ltd.

UniPSN 10-300 chromatographic medium was from Suzhou Nanomicro Technology Co., Ltd.

### Main equipment

| Equipment Name | Specification and Model | Manufacturer |
|---|---|---|
| High Speed Refrigerated Centrifuge | H5-25KR | Hunan Kecheng Instrument and Equipment Co., Ltd |
| Membrane Package Ultrafiltration System | 0.5m² | Hangahou Cobetter Filtration Equipment Co., Ltd |
| High Performance Liquid Chromatograph | DAC150 | Jiangsu Hanbon Science & Technology Co., Ltd |

### Main buffer solutions

| Name and Ingredient of Buffer Solution |
|---|
| Washing solution 1 (0.5M NaOH) |
| Preservation solution 1 (100mM NaOH) |
| Diluent (30% acetonitrile~5 mM citric acid pH 5.0) |
| A1 solution (30% acetonitrile 0.1%TFA) |
| A2 solution (20% acetonitrile 0.1 %TFA) |
| B solution (99% acetonitrile 0.1 %TFA) |
| Dissolving solution (5 mM histidine pH 6.0) |
| Ultrafiltration replacement solution (5 mM histidine pH 5.0) |

### Step 1. Dissolution, filtration and rough purification of saponin

### 1.1. Dissolution and filtration of saponin

Feed amount: saponin 200 g, diluent 5 L.

Saponin was dissolved in the diluent (30% acetonitrile ~5 mM citric acid, with pH 5.0) according to 1:25 (W/V), and filtered by a 1.0 + 0.45 µm filter. After filtration, 5.11-5.15 L of **saponin filtered sample** was obtained.

### 1.2. Reverse phase rough purification of saponin solution

(1) Pre-processing: connecting a chromatographic column and a liquid chromatography system, controlling an A1 solution to enter an A pump port and a B solution to enter a B pump port respectively, a system linear flow rate being 100-140 cm/h, 100% B rinsing, followed by 100% A balancing.
(2) Loading: loading the saponin filtered sample (about 5 L) obtained in step 1 on the UniPSN 30-300 chromatographic medium, in a loading amount of 30-60 mg/ml, a linear flow rate being 90-360 cm/h, after finishing the loading, rinsing with the A1 solution (4.5 L), and then rinsing impurities with 2.5% B in turn.
(3) Elution: performing linear gradient elution of target peak with 2.5% B to 21.25±5.00% B, collecting samples periodically to undergo purity detection, and merging samples with purity higher than 30%, to obtain 4.7-5.5 L of roughly purified saponin solution.

Amounts of materials used in the loading and elution in the step of reverse phase rough purification are shown in **Table 8**.

**Table 8. Reverse Phase Rough Purification of Saponin**

| Operation Step | Material Name | Feed Amount |
|---|---|---|
| Loading | Saponin Filtered Sample | 5.11∼5.15L |
| Column Rinsing | 100%A1 | 7.8L |
| Elution | 2.50%B | 11.6L |
| | 2.50%B~21.25±5.00%B | 35.0L |

### Step 2. Fine purification of saponin

### 2.1. First step of reverse phase fine purification of saponin solution

(1) Pre-processing: connecting the chromatographic column and the liquid chromatography system, controlling an A2 solution to enter the A pump port and the B solution to enter the B pump port respectively, a system linear flow rate being 100-140 cm/h, 100% B rinsing, followed by 100% A balancing.
(2) Loading: diluting the roughly purified saponin solution in water, controlling acetonitrile concentration to be 22-28%, and loading the diluted saponin solution on the UniPS 10-300 chromatographic medium (loading the roughly purified saponin solution having been diluted in total, in a loading amount of about 10-50 mg/ml), a linear flow rate being 100-140 cm/h; after finishing the loading, rinsing with the A2 solution, and then rinsing impurities with 10% B in turn.
(3) Elution: performing linear gradient elution of target peak with 10% B to 25±5% B, collecting samples periodically to undergo purity detection, and merging samples with purity higher than 60%, to obtain 4.94-5.30 L of two-step purified saponin sample solution.

Amounts of materials used in the loading and elution in the first step of reverse phase fine purification are shown in**Table 9.**

**Table 9. First Step of Reverse Phase Fine Purification of Saponin**

| Operation Step | Material Name | Feed Amount |
|---|---|---|
| Loading | Roughly purified saponin solution | 4.7∼5.5L |
| | Water | 3.2~3.7L |
| Column Rinsing | 100% A2 | 4.4L |
| Elution | 10% B | 4.4L |
| | 10%B~25+5%B | 66.2 L |

### 2.2. Second step of reverse phase fine purification of saponin solution

(1) Pre-processing: connecting the chromatographic column and the liquid chromatography system, controlling the A2 solution to enter the A pump port and the B solution to enter the B pump port respectively, a system linear flow rate being 100-140 cm/h, 100% B rinsing, followed by 100% A balancing.
(2) Loading: diluting the two-step purified saponin sample solution in water, controlling acetonitrile concentration to be 22-28%, and loading the diluted saponin sample solution on the UniPS 10-300 chromatographic medium (loading the diluted sample in total, in a loading amount of about 10~50 mg/ml), a linear flow rate being 100~140 cm/h; after finishing the loading, rinsing with the A2 solution, and then rinsing impurities with 10% B in turn.
(3) Elution: performing linear gradient elution of target peak with 10% B to 25±5% B, collecting samples periodically to undergo purity detection, and merging samples with purity higher than 80%, to obtain 1.76-2.30 L, as three-step purified saponin solution.

Amounts of materials used in the loading and elution in the second step of reverse phase fine purification are shown in following **Table 10**.

**Table 10. Second Step of Reverse Phase Fine Purification of Saponin**

| Operation Step | Material Name | Feed Amount |
|---|---|---|
| Loading | Two-step purified saponin solution | 4.94∼5.30L |
| | Water | 2.2~2.4L |
| Column Rinsing | 100% A2 | 4.4L |
| Elution | 10% a | 4.4L |
| | 10%B~25±5%B | 66.2L |

### 2.3. Third step of reverse phase fine purification of saponin solution

(1) Pre-processing: connecting the chromatographic column and the liquid chromatography system, controlling the A2 solution to enter the A pump port and the B solution to enter the B pump port respectively, a system linear flow rate being 100-140 cm/h, 100% B rinsing, followed by 100% A balancing.
(2) Loading: diluting the three-step purified saponin sample solution in water, controlling acetonitrile concentration to be 22-28%, and loading the diluted saponin sample solution on the UniPS 10-300 chromatographic medium (loading the diluted sample in total, in a loading amount of about 10~50 mg/ml), a linear flow rate being 100~140 cm/h; after finishing the loading, rinsing with the A2 solution, and then rinsing impurities with 10% B in turn.
(3) Elution: performing linear gradient elution of target peak with 10% B to 25±5% B, collecting samples periodically to undergo purity detection, and merging samples with purity higher than 95%, to obtain 1.41-1.81 L, as four-step purified saponin sample solution.

Amounts of materials used in the loading and elution in the third step of reverse phase fine purification are shown in following **Table 11.**

**Table 11. Third Step of Reverse Phase Fine Purification**

| Operation Step | Material Name | Feed Amount |
|---|---|---|
| Loading | Three-step purified saponin solution | 1.76~2.30L |
| | Water | 0.8∼1.1L |
| Column Rinsing | 100%A2 | 4.4 L |
| Elution | 10%B | 4.4 L |
| | 10%B~25±5%B | 66.2L |

### Step 3. Precipitation and re-dissolution, ultrafiltration replacement, and subpackaging for the saponin

### 3.1. Step of precipitation and re-dissolution

(1) To the four-step purified saponin sample solution (1.4-1.8 L) prepared in Example 2, adding water of 2 times volume and uniformly mixing them, placing the mixture at room temperature for not less than 30 min, then centrifuging at 9000-11500 g and 2-8°C for 10 min, and collecting precipitate.
(2) To the precipitate adding a dissolving solution 1.5-3.0 L, and stirring and dissolving the mixture, to obtain 1.5-3.0 L of sample with organic solvent removed.

### 3.2. Step of ultrafiltration replacement

(1) Rinsing an ultrafiltration membrane with an ultrafiltration replacement solution (5 mM histidine pH 5.0); ultrafiltration-concentrating the saponin sample (about 1.5-3.0 L) with organic solvent being removed obtained by precipitation and re-dissolution in step 1 of the present example to 0.91-0.93 L; and replacing with 5 kD membrane package for 10 times, transmembrane pressure being 0.2-0.4 bar.
(2) Washing-filtering with the ultrafiltration replacement solution for not less than 8 times volume, to obtain 0.91-0.93 L of saponin ultrafiltration sample.

After the 5 kD membrane is used, yield is more than 70%, improved over conventional 10 kD ultrafiltration replacement membrane package.

### 3.3. Subpackaging

The saponin ultrafiltration sample having undergone the ultrafiltration replacement is filtered and sterilized with a 0.45 + 0.2 µm filter, to obtain the QS21 adjuvant solution of the present invention, which is subpackaged and stored at a temperature below -70°C.

### Step 4. Key process quality detection

### 4. 1. Detection method

Intermediate samples of various steps in three batches of pilot production were detected by RP-HPLC, and impurity peak removal effects in each chromatography step were compared and analyzed. Chromatographic conditions are shown in **Table 12**.

**Table 12. Chromatography Step**

| Chromatographic Column | Waters, ACQUITY UPLC CSHTM, C18 2.1 × 150 mm, 1.7µm |
|---|---|
| Column Temperature | 40°C |
| Automatic Sampler Temperature | 8°C |
| UV-detector Wavelength | 214nm |
| Mobile Phase | Mobile phase A: deionized water, containing 0.5% acetic acid |
| | Mobile phase B: 100% acetonitrile |

### 4.2. Efficiency of removing main impurities

The samples obtained by the reverse phase rough purification and the first step of reverse phase fine purification had many impurity peaks, among which residence time of QS-21 main peak was at 22.622 min, and three peaks whose residence time was at 20.376 min (No. 1 impurity), 20.734 min (No. 2 impurity), and 23.109 min (No. 3 impurity) were the main impurity peaks.

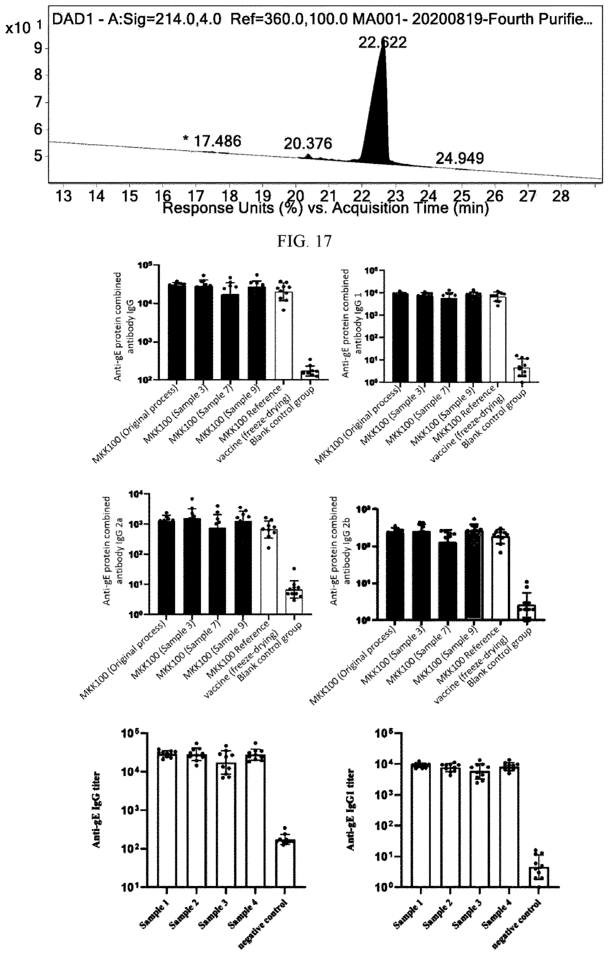

A detection chromatogram of the QS-21 sample after the third step of reverse phase fine purification (after the four-step purification) is shown in **FIG. 17**.

As the samples obtained by the reverse phase rough purification and the first step of reverse phase fine purification had many impurity peaks, the effect of chromatographic impurity removal is calculated only for results after the second step of reverse phase fine purification (after three-step purification) and the third step of reverse phase fine purification (after four-step purification). Main impurity removal effects of the four-step purification and the three-step purification are compared in **Table 13.**

**Table 13. Comparison of Main Impurity Removal Effects of Four-step Purification Versus Three-step Purification**

| - | - | Impurity Peak No. | | |
|---|---|---|---|---|
| - | QS-21 Batch No. | 1 | 2 | 3 |
| Impurity Peak Residence Time | - | 20.376min | 20.734min | 23.109min |
| | | Removal Rate | Removal Rate | Removal Rate |
| Step 4: Second step of reverse phase fine purification | MA001-202008-10 | 61.71% | 84.00% | 63.53% |
| | MA001-202008-15 | 55.38% | 64.25% | 56.76% |
| | MA001-202008-19 | 54.73% | 45.04% | 71.94% |
| Step 5: Third step of reverse phase fine purification | MA001-202008-10 | 100.00% | 83.03% | 90.00% |
| | MA001-202008-15 | 77.02% | 64.20% | 89.91% |
| | MA001-202008-19 | 57.00% | 59.18% | 89.45% |

Removal rate = total amount of impurity peaks of chromatographically harvested samples / total amount of impurity peaks of loaded sample in chromatography, where total amount of impurity peaks = (RP-HPLC detected UV peak area / feed volume) * sample volume in chromatography step.

### 4.3. QS-21 purity and yield

QS-21 purity was assessed by the same RP-HPLC. The results are shown in **Table 15**.

**Table 15. QS-21 Purity Detection Results**

| Intermediate Type | Impurity Detection Result | MA001-20200810 | | MA001-20200815 | | MA001-20200819 | |
|---|---|---|---|---|---|---|---|
| | | Prechromatography | Postchromatography | Prechromatography | Postchromatography | Prechromatography | Postchromatography |
| QS21 from Reverse Phase Rough Purification | Purity | 8.0% | 46.1% | 8.0% | 48.5% | 8.1% | 48.4% |
| QS21 from First Step of Reverse Phase Fine Purification | | 46.1% | 67.5% | 48.5% | 71.1% | 48.4% | 62.4% |
| QS21 from Second Step of Reverse Phase Fine Purification | | 67.5% | 85.0% | 71.1% | 86.9% | 62.4% | 82.4% |
| QS21 from Third Step of Reverse Phase Fine Purification | | 85.0% | 95.7% | 86.9% | 95.0% | 82.4% | 94.1% |

QS-21 yield was detected by the same RP-HPLC. Results are shown in **Table 16**.

**Table 16. QS-21 Yield Detection Results**

| Chromatography Step | MA001-20200810 | MA001-20200815 | MA001-20200819 |
|---|---|---|---|
| Reverse Phase Rough Purification | 76.77% | 75.37% | 80.60% |
| First Step of Reverse Phase Fine Purification | 82.64% | 83.21% | 71.87% |
| Second Step of Reverse Phase Fine Purification | 37.87% | 51.13% | 60.10% |
| Third Step of Reverse Phase Fine Purification | 80.46% | 53.78% | 63.17% |

### Step 5. QS-21 functional verification

QS-21 samples prepared by the preceding steps were prepared into the MA105 adjuvant system, herpes zoster vaccine, and anti-tumor vaccine product according to the methods in the preceding Examples 1-11, and their activity was compared with that of existing commercial QS-21 products.

### 5.1. Herpes zoster vaccine product

QS-21 prepared in the present example and purchased QS-21 were formulated into gE/composite adjuvant candidate vaccines, respectively, and immunogenicity differences between the two formulations were compared.

Test method: at the start, pre-immunization was carried out first, then mice were divided into 3 groups, 10 mice in each test group, 5 mice in gE/buffer solution control group.

Pre-immunization method: female C57BL/6 mice aged 5-6 weeks were subcutaneously administered with 104-5 TCID50 VZV attenuated viruse.

5 weeks after pre-immunization, the mice were intramuscularly injected on days 0 and 28, 0.05 ml per mouse each time.

14 days after the second vaccination, blood was collected from the mice, and binding antibodies in serum of individuals were detected by ELISA.

Spleens were isolated, and the frequency of CD4⁺ T cells generating cytokines (IL-2/IFN-γ) was evaluated by intracellular cytokine staining and flow cytometry.

Experimental results show that:
(1) After vaccination with the herpes zoster vaccines formulated with QS-21 from two different sources, the binding antibody titer had no significant difference; and
(2) Both vaccine preparations induced the generation of (IL-2/IFN-γ) CD4⁺T-cell responses.

It can be seen that the gE/composite candidate vaccines formulated with the QS-21 prepared by the method procedures in the present example and the purchased QS-21 have no significant difference in inducing immune responses in mice.

Finally, the same therapeutic activity results are also verified in the MA105 adjuvant-based anti-tumor vaccines and antigen-free therapeutic agents (Examples 8-10).

Finally, it should be noted that the above examples are only intended to help those skilled in the art understand the essence of the present invention, and are not intended to limit the scope of protection of the present invention.

## Claims

1. An MA105 adjuvant system, **characterized by** comprising:
(1) QS-21, 50-300 µg/ml;
(2) Poly I:C, 400-3000 µg/ml; and
(3) lipid molecules constituting a delivery system, wherein
the delivery system is a mixture of cationic and neutral liposomes.

2. The immunologic adjuvant according to claim 1, wherein the adjuvant comprises:
(1) QS-21, 50-200 µg/ml, preferably 80-120 µg/ml;
(2) Poly (I:C), 400-3000 µg/ml, preferably 780-1600 µg/ml; and
the lipid molecules are:
(3) DOTAP, 70-560 µg/ml, preferably 140-280 µg/ml;
(4) DOPC, 500-4000 µg/ml, preferably 1200-2500 µg/ml; and
(5) cholesterol, 175-1400 µg/ml, preferably 350-600 µg/ml.

3. A preparation method for the MA105 adjuvant system according to claim 1 or 2, **characterized by** comprising the following steps:
(1) preparing the cationic liposomes and the neutral liposomes, respectively; and
(2) mixing the QS-21, the poly (I:C), the cationic liposomes, and the neutral liposomes and stirring well to obtain the MA105 adjuvant system, wherein
a preparation method for the cationic liposomes is:
(1) formulating 1,2-dioleoyl-3-trimethyl ammonium chloride propane (DOTAP), dioleoyl phosphatidylcholine (DOPC) and cholesterol according to a mass ratio of 2:2: 1; and
(2) dispersing three lipid ingredients uniformly, and then preparing the liposomes by an ethanol injection method, a film dispersion method, an ultrasonic dispersion method, a reverse evaporation method; and
a preparation method for the neutral liposomes is:
(1) formulating dioleoyl phosphatidylcholine (DOPC) and cholesterol according to a mass ratio of 4:1; and
(2) dispersing lipid ingredients uniformly, and then preparing the liposomes by an ethanol injection method, a film dispersion method, an ultrasonic dispersion method, a reverse evaporation method.

4. The method according to claim 3, wherein preparation steps of the cationic liposomes are as follows:
(1) dissolving the three lipid ingredients in ethanol, and shearing on line with a water phase for 1-3 times;
(2) buffer replacement by ultrafiltration using a sucrose-containing buffer solution, concentration multiple being 2-4 times and washing-filtering 6-8 times;
(3) extruding by a filter membrane with a pore size of 200 nm, cyclically performing multiple times of extrusion, and controlling particle size homogeneity;
(4) extruding by a filter membrane with a pore size of 100 nm, cyclically performing multiple times of extrusion, and controlling particle size homogeneity; and
optionally, (5) filtering and sterilizing by a sterile filter, wherein
physical and chemical parameters of the cationic liposome are:
average particle size (D50): 30-90 nm;
solution pH: 4.8-7.0, preferably 4.8-6.0; and
Zeta potential: 40-72 mV (pH 6.5).

5. The method according to claim 3, wherein preparation steps of the neutral liposomes are as follows:
(1) dissolving two lipid ingredients in ethanol, and shearing on line with a water phase for 1-3 times;
(2) filtering by a filter membrane with a pore size of 200 nm, cyclically performing multiple rounds of extrusion, and controlling particle size homogeneity;
(3) filtering by a filter membrane with a pore size of 100 nm, cyclically performing multiple rounds of extrusion, and controlling particle size homogeneity;
(4) buffer replacement via ultrafiltration using a sucrose-containing buffer solution 3-6 times and washing-filtering 6-8 times; and
optionally, (5) sterilizing by filtration, wherein
physical and chemical parameters of the neutral liposomes are:
average particle size (D50): 80-140 nm; and
solution pH: 5.5-7.0, preferably 6.4-6.6.

6. Use of the MA105 adjuvant system according to claim 1 or 2 in a preparation of a vaccine preparation, **characterized in that** the vaccine preparation comprises
a therapeutically effective amount of antigen,
the MA105 adjuvant system, and
indispensable pharmaceutical auxiliary materials, wherein
the pharmaceutical auxiliary materials comprise polysorbate 80, histidine, sodium dihydrogen phosphate, sucrose, or sodium chloride.

7. A method for preparing a recombinant herpes zoster vaccine preparation with the MA105 adjuvant system according to claim 1 or 2, **characterized in that** the method comprises steps as follows:
(1) to a purified antigen solution adding sucrose, histidine and Tween-80 according to a formula of an antigen buffer solution, and regulating pH to obtain a primary antigen liquid, wherein an antigen in the primary antigen liquid is a gE protein antigen having a sequence set forth in SEQ ID NO. 1;
(2) mixing the primary antigen liquid and the MA105 adjuvant system and stirring uniformly to obtain a semi-finished product; and
(3) subpackaging the semi-finished product to obtain a finished product of the recombinant herpes zoster vaccine preparation.

8. The method according to claim 7, wherein in terms of per milliliter, the recombinant herpes zoster vaccine preparation comprises:
(1) the gE protein antigen having a sequence set forth in SEQ ID NO. 1, with a content of 95-110 µg/ml;
(2) a pharmaceutical auxiliary material combination for maintaining stability of the gE protein antigen, the pharmaceutical auxiliary material combination comprising: polysorbate 80, 450-550 µg/ml; histidine, 200-950 µg/ml; and sucrose, 52-55 mg/ml;
(3) the MA105 immunologic adjuvant system, comprising:
1) DOTAP, 140-215 µg/ml;
2) DOPC, 1200-2200 µg/ml;
2) cholesterol, 320-550 µg/ml;
3) QS-21, 80-110 µg/ml; and
4) Poly (I:C), 780-830 µg/ml; and
(4) other pharmaceutical auxiliary materials, comprising:
1) sodium dihydrogen phosphate, 0-800 µg/ml; and
2) sodium chloride, 2.5-6 mg/ml.

9. A recombinant herpes zoster vaccine prepared by the method according to claim 7 or 8.

10. The recombinant herpes zoster vaccine preparation according to claim 9, wherein
an osmotic pressure of the vaccine preparation is 210-350 mOsmol/kg;
an average particle size of the vaccine preparation is 100-300 nm, preferably 130-200 nm; and
pH of the vaccine preparation is 6.0-7.0.

11. Use of the recombinant herpes zoster vaccine preparation according to claim 9 or 10 in a treatment or prevention of diseases caused by varicella zoster virus (VZV), **characterized in that** the treatment is single treatment or combined treatment with other drugs or vaccines.

12. An anti-tumor vaccine product, **characterized in that** the anti-tumor vaccine product comprises:
(1) a therapeutically effective amount of tumor antigen, the tumor antigen being a recombinant polypeptide antigen;
(2) the MA105 adjuvant system according to claim 1 or 2; and
(3) indispensable pharmaceutical auxiliary materials, wherein
preferably, the anti-tumor vaccine product is a subcutaneous or intramuscular injection type vaccine;
preferably, the tumor antigen is an HPV antigen having an amino acid sequence set forth in SEQ ID NO. 2 or an HPV antigen having an amino acid sequence set forth in SEQ ID NO. 3;
preferably, the pharmaceutical auxiliary materials comprise: a stabilizer, a freeze-thawing protective agent, and an osmotic pressure regulator; and
more preferably, the pharmaceutical auxiliary materials comprise: polysorbate 80, sucrose, sodium chloride, histidine, and sodium dihydrogen phosphate.

13. An anti-tumor preparation, **characterized by** comprising:
(1) a therapeutically effective amount of the MA105 adjuvant system according to claim 1 or 2; and
(2) indispensable pharmaceutical auxiliary materials, wherein
preferably, the anti-tumor preparation is an intratumoral injection preparation or a tumor in-situ vaccine;
preferably, the tumor is melanoma, colorectal cancer or lung cancer; and
preferably, the pharmaceutical auxiliary materials comprise: a stabilizer, a freeze-thawing protective agent, and an osmotic pressure regulator; and more preferably, the pharmaceutical auxiliary materials comprise: polysorbate 80, sucrose, and sodium chloride.

14. Use of the MA105 adjuvant system according to claim 1 or 2 or a preparation comprising the MA105 adjuvant system in a treatment of tumors, **characterized in that** the treatment is single treatment or combined treatment; and
the combined treatment is a therapeutically effective amount of the MA105 adjuvant system that is used in combination with any other drug selected from the group consisting of:
(1) a cytotoxic chemotherapeutic drug;
(2) an antibody anti-tumor drug;
(3) an oncolytic peptide and oncolytic virus drug; and
(4) a cell therapy preparation, preferably Car-T cell therapy preparation.

15. A preparation method for preparing Quillaja saponaria saponin 21 (QS-21) immunologic adjuvant from a semi-purified saponin raw material, **characterized in that** the method comprises steps as follows:
(1) saponin dissolution and filtration, comprising
dissolving the saponin raw material in a diluent, and then removing undissolved substances by filtration;
(2) reverse phase rough purification, comprising
carrying out reverse phase rough purification on a saponin filtered sample solution obtained in step (1) according to the following steps:
1) loading: loading on a UniPSN 30-300 chromatographic medium; and
2) elution: performing linear gradient elution of target peak with 2.5% B to 21.25±5.00% B, periodically collecting high-purity samples, and merging roughly purified saponin solution;
(3) reverse phase fine purification, comprising
reverse phase fine purification of the roughly purified saponin solution obtained in step (2) according to steps as follows:
1) loading: diluting sample solution in water to acetonitrile concentration of 22-28%, and loading on a UniPS 10-300 chromatographic medium;
2) elution: performing linear gradient elution of target peak with 10% B to 25±5% B, periodically collecting high-purity samples, and merging; and
3) making a merged sample repeat the above 1)-2) steps for 1-3 times, preferably 2 times;
(4) a step of precipitation and re-dissolution, comprising
1) to purified saponin sample solution prepared in step (3), adding water of 2 times volume and uniformly mixing, placing at room temperature, then centrifuging and collecting a precipitate; and
2) to the precipitate adding a solution, stirring and dissolving, to obtain a sample with organic solvent being removed, preferably, an amount of the dissolving solution added being the same as that of the purified saponin sample solution prepared in step (3); and
(5) buffer replacement by ultrafiltration, comprising
1) ultrafiltration-concentrating a saponin sample solution obtained after precipitation and re-dissolution in step (4) with an ultrafiltration replacement solution, to obtain a concentrated QS-21 adjuvant solution, preferably, ultrafiltration-concentrating to 30%-50% of the original volume, wherein
in the above steps (1)-(5),
the diluent is a water solution containing 30% acetonitrile and 5 mM citric acid, pH 5.0;
B solution is a water solution containing 99% acetonitrile and 0.1% TFA;
the dissolving solution is a water solution containing 5 mM histidine, pH 6.0; and
the ultrafiltration replacement solution is a water solution containing 5 mM histidine, pH 5.0.

16. The method according to claim 15, wherein in the semi-purified saponin raw material, a QS-21 content is not lower than 8%, a moisture content is not higher than 10%, and an ash content is not higher than 3%.

17. The method according to claim 15 or 16, wherein
in step (1), the saponin raw material is dissolved according to 1:25 (W/V), and then filtered by a 1.0 + 0.45 µm filter;
in step (2), a loading amount is 30-60 mg/ml, and a linear flow rate is 90-360 cm/h; after finishing the loading, A1 solution is used for rinsing, and impurities are rinsed with 2.5% B solution; and in the gradient elution, samples with purity higher than 30% are collected and merged;
in step (3), a loading amount is 10-50 mg/ml, after finishing the loading, A2 solution is used for rinsing, and impurities are rinsed with 10% B, wherein samples with purity higher than 60% are collected in first reverse phase fine purification, samples with purity higher than 80% are collected in second reverse phase fine purification, samples with purity higher than 90% are collected in third reverse phase fine purification, and samples with purity higher than 95% are collected in fourth reverse phase fine purification;
in step (4), the mixture is placed at room temperature for not less than 30 min, and centrifuging parameters are 9000-11500 g, 2-8 °C and 10 min; and
in step (5), ultrafiltration parameters are: using 5 kD membrane package, transmembrane pressure of 0.2-0.4 bar, 10 times of replacement, and washing-filtering with the ultrafiltration replacement solution for not less than 8 times volume,
wherein
the A1 solution is a water solution containing 30% acetonitrile and 0.1% TFA; and
the A2 solution is a water solution containing 20% acetonitrile and 0.1% TFA.
